(19) Europäisches Patentamt
European Patent Office
Office européen des brevets

(11) **EP 3 767 631 A1**

(12) **EUROPEAN PATENT APPLICATION**

(43) Date of publication:
**20.01.2021 Bulletin 2021/03**

(51) Int Cl.:
***G16H 20/10*** *(2018.01)* ***G01N 33/52*** *(2006.01)*

(21) Application number: **19187294.4**

(22) Date of filing: **19.07.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Vrije Universiteit Brussel**
**1050 Brussel (BE)**
• **Universiteit Antwerpen**
**2000 Antwerpen (BE)**

(72) Inventors:
• **MEULEBROECK, Wendy**
**1050 Brussels (BE)**

• **OTTEVAERE, Heidi**
**1050 Brussels (BE)**
• **THIENPONT, Hugo**
**1050 Brussels (BE)**
• **GOSSENS, Herman**
**1730 Asse (BE)**
• **MOONS, Pieter**
**2200 Hallaar (BE)**
• **VAES, Kristof**
**2811 Leest (BE)**
• **DE BOECK, Muriel**
**2640 Mortsel (BE)**
• **BAELUS, Christiaan**
**2600 Werpen (BE)**

(74) Representative: **Brantsandpatents bvba**
**Pauline Van Pottelsberghelaan 24**
**9051 Ghent (BE)**

(54) **METHOD FOR EVALUATING ANTIBIOTIC SUSCEPTIBILITY**

(57) A method for determining a minimum inhibitory concentration (MIC) to an antimicrobial drug for a bacteria culture in a container , the method comprising calculating for each well a third derivative of said detected intensity in a first time frame and in a second time frame, said second time frame being subsequent, preferably directly subsequent, to said first time frame; comparing said third derivative of the detected intensity in both said time frames to one or more threshold values; determining a first MIC value based on said comparison of said third derivative in said first time frame to said one or more threshold values, and determining a second MIC value based on said comparison of said third derivative in said second time frame to said one or more threshold values; and determining from said first and said second MIC values the MIC value of said bacteria culture.

EP 3 767 631 A1

**Description**

**FIELD OF THE INVENTION**

The present invention relates to a method for determining a minimum inhibitory concentration to an antimicrobial drug for a bacteria culture.

**BACKGROUND**

EP 1 160 564 B1 relates to a system and method for analysing samples, such as biological samples, to determine the susceptibility of the samples to antimicrobial materials, such as antibiotics. More particularly, it relates to a system and method which takes a plurality of optical readings of a biological sample contained in sample wells of a sample test panel having various types and concentrations of antimicrobial materials therein and, based on these readings, determines the respective minimum inhibitory concentrations (MICs) at which the respective antimicrobial materials will inhibit growth of the sample. The determination of MIC is based on redox and turbidity values.

WO 2014 145 899 A1 relates to systems and methods for rapid determination of microorganism growth and antimicrobial agent susceptibility and/or resistance. More particularly, the document relates to a computer-based system configured to analyse microorganism information. A first value associated with an attribute of a microorganism is determined by the computer-based system, based on first information obtained from a microorganism detection system. A second value associated with an attribute of the microorganism is determined, optionally by the computer-based system, based on second information obtained from a microorganism detection system. A growth rate is determined by reference to the first value and the second value. The growth rate is compared to a control growth rate.

US 9 834 808 B2 relates to determination of antibiotic susceptibility of microbial infections. The invention is based in part upon non-specific surface binding assays. Furthermore, the present invention may include signaling agents (e.g., Europium compounds) that are bound to microorganisms non-specifically rather than specifically. Also, the possibility of using signal amplification is discussed. Europium formulations as chemical moiety may be used. The method includes steps of incubating a liquid suspension of microorganisms in the presence of an antimicrobial and a signalling agent, which is capable of binding to a surface of the microorganisms; separating the microorganisms bound by the signalling agent from the unbound signalling agent; and determining signal levels associated with the microorganisms as compared to one or more controls.

US 20180 305 732 A1 relates to methods for identifying the susceptibility, tolerance or resistance of a microorganism to one or more antimicrobial agents, and the level of tolerance. The method comprises exposing the microorganism grown on a plate surface to one or more antimicrobial agents and to at least one growth promoting agent.

There still remains a need in the prior art to simplify susceptibility testing and make the testing less time consuming and more efficient for the administrator. Further, it is known when antibiotic susceptibility is tested it is also important to test for heteroresistancy of a biological sample. Therefor, there also still remains a need to more quickly determine the minimum inhibitory concentration together with the heteroresistancy of a bacteria culture.

**SUMMARY OF THE INVENTION**

The present invention and embodiments thereof serve to provide a solution to one or more of above-mentioned disadvantages. To this end, the present invention relates to a method according to claim 1.

The method according to the present invention is advantageous for fast and accurate determination of the susceptibility of a bacteria culture to a microbial drug. The applicant has noticed that by employing a third derivative of the growth curve of a bacteria culture, the minimum inhibitory concentration can be accurately detected through a simple experimental setup, whereby the setup only requires a bacterial culture, a growth medium for the bacteria culture, a microbial drug and a technical setup (e.g. a light source in combination with a photodetector or spectrophotometer system with a fluidic chip) for measuring the bacteria growth in time. Compared to other methods employing a technological setup, the present method is beneficial for reducing the time and the cost of testing the bacteria susceptibility. Moreover, the applicant has noticed that using the method according to the present invention, the minimum inhibitory concentration for some bacteria cultures can be determined already after 1 to 3 hours of bacteria growth.

The method according to an embodiment of the invention is particularly suitable for determining a minimum inhibitory concentration to an antimicrobial drug for a bacteria culture and the presence of heteroresistancy.

Further advantages, features, and examples of the present invention are disclosed in the detailed description.

**DESCRIPTION OF FIGURES**

The following description of the figures of specific embodiments of the invention is merely exemplary in nature and is not intended to limit the present teachings, their application or uses. Throughout the drawings, corresponding reference numerals indicate like or corresponding parts and features.

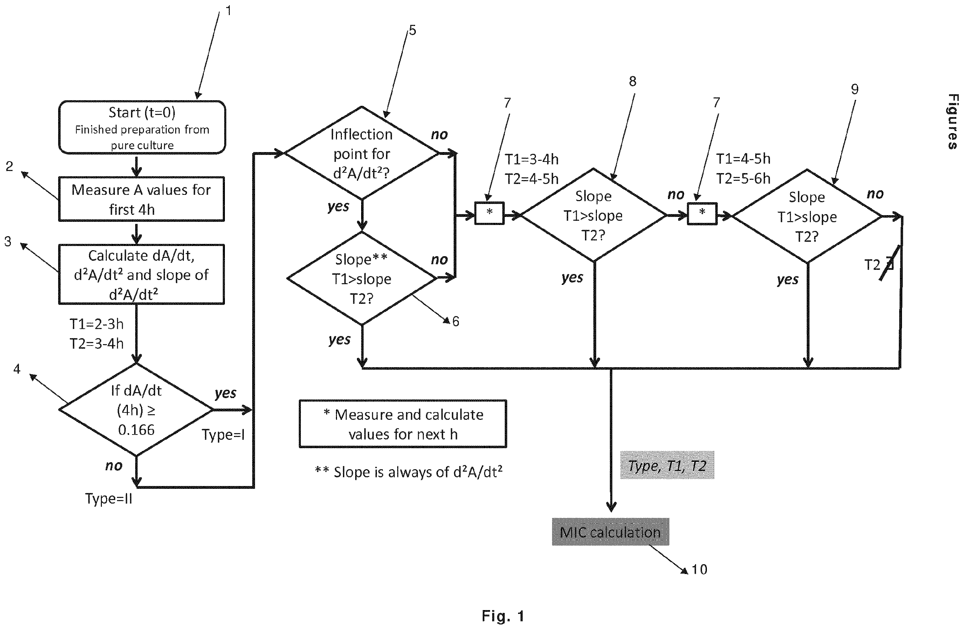

**Figure 1** shows a diagram of a method for determining a first time frame, a second time frame and the type bacteria culture in accordance with an embodiment of the disclosure.

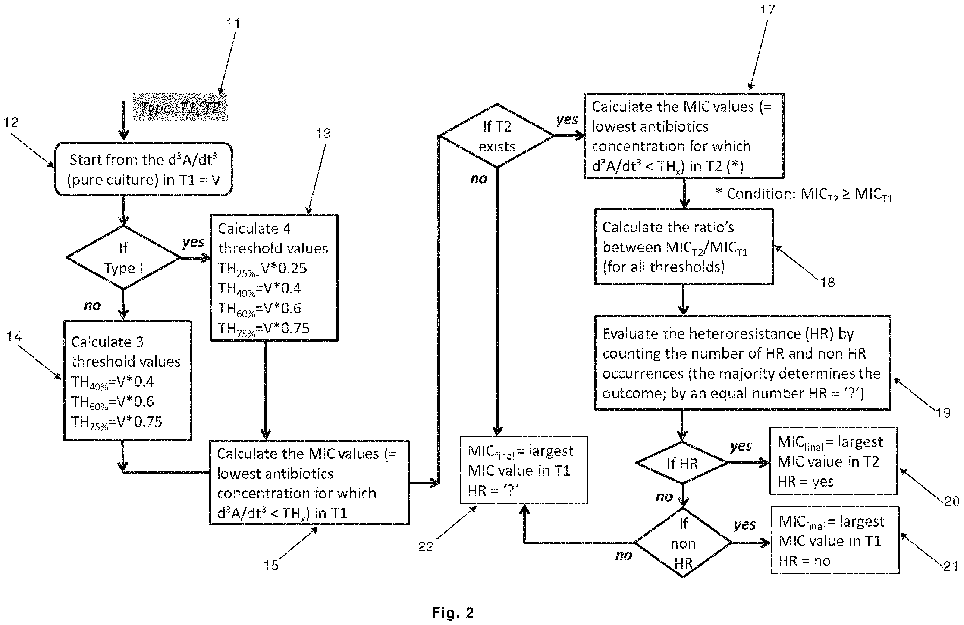

**Figure 2** shows a diagram of a method for determining a minimum inhibitory concentration of a bacteria culture to a chemical substance in accordance with an embodiment of the disclosure.

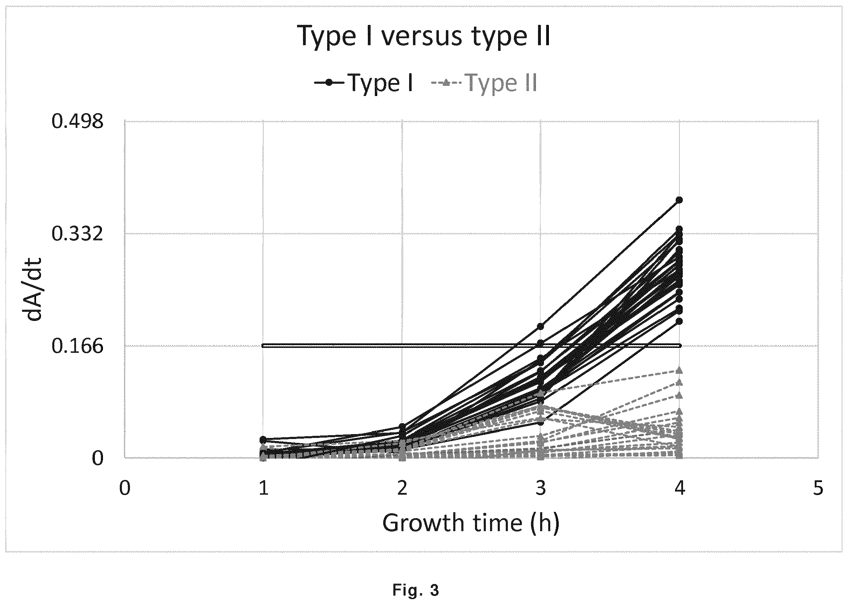

**Figure 3** shows a graph of a first derivative of measured light intensities, represented by calculated absorbance values, over the growth time for experiments testing a minimum inhibitory concentration of different bacteria cultures to a chemical substance.

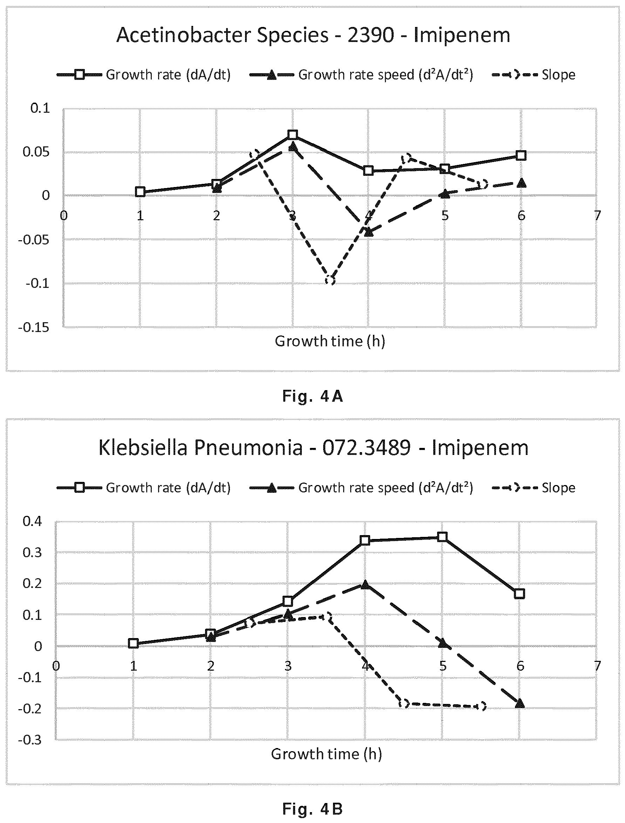

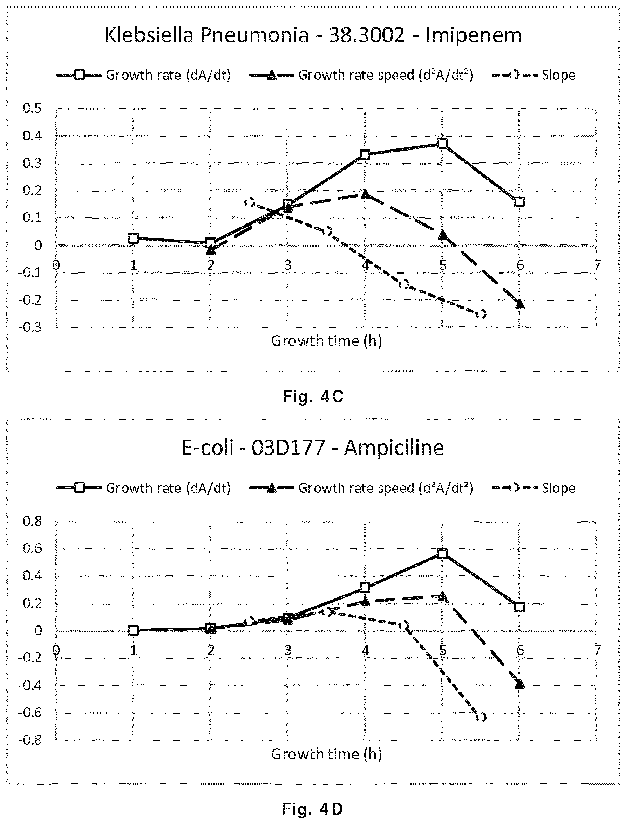

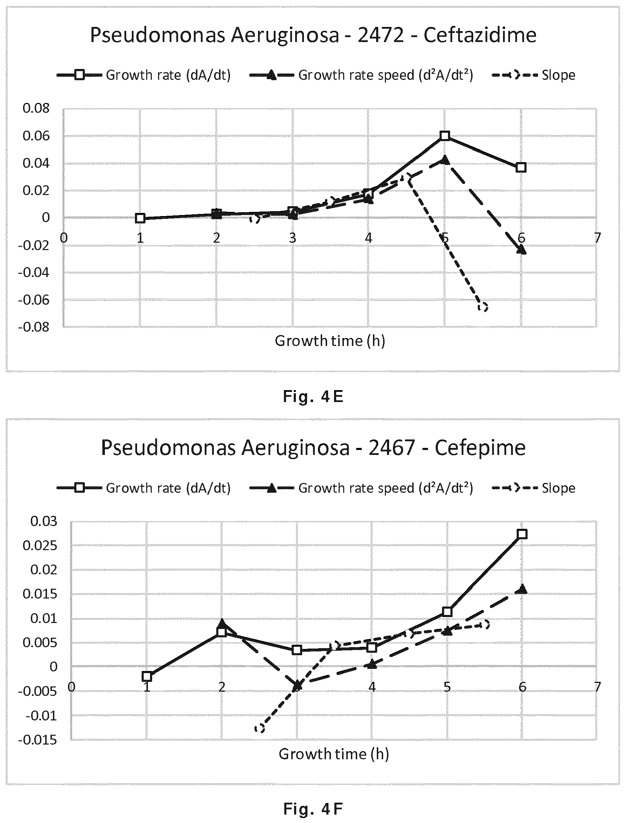

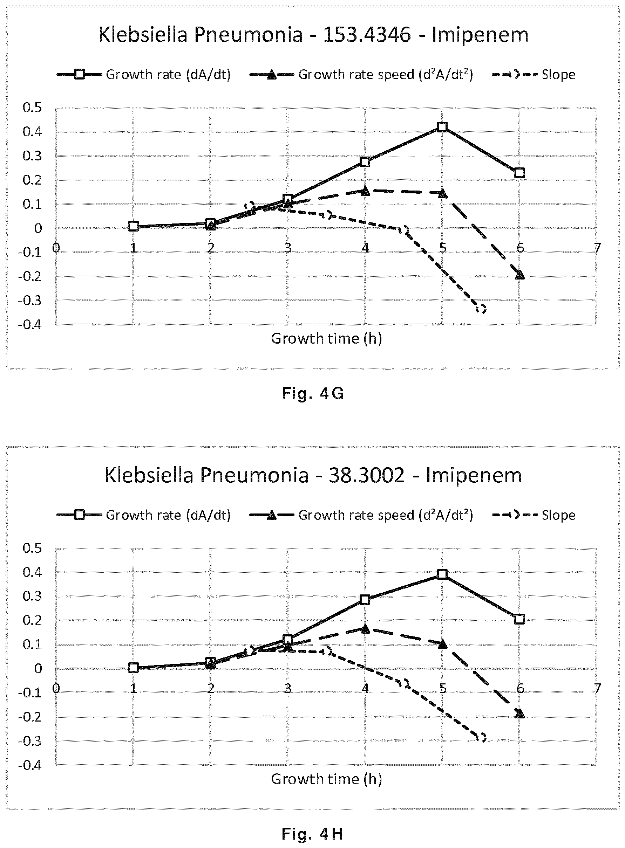

**Figures 4A, 4B, 4C, 4D, 4E, 4F, 4G and 4H** each represent a graph of a first, second, and third derivative of measured light intensities, represented by calculated absorbance values, over the growth time for a experiment testing a minimum inhibitory concentration and heteroresistancy of a bacteria cultures to a chemical substance in accordance with an embodiment of the disclosure.

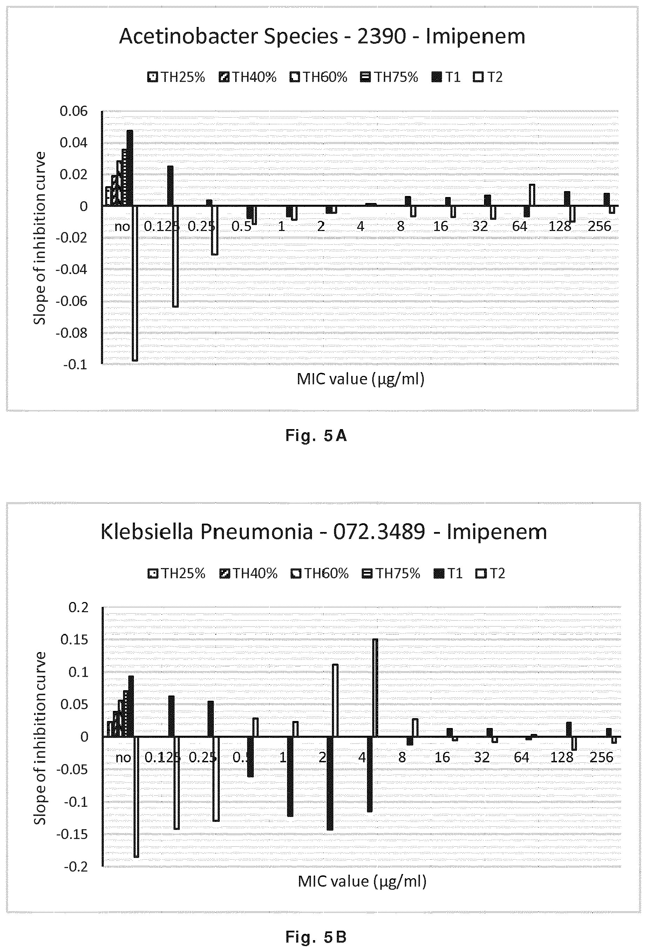

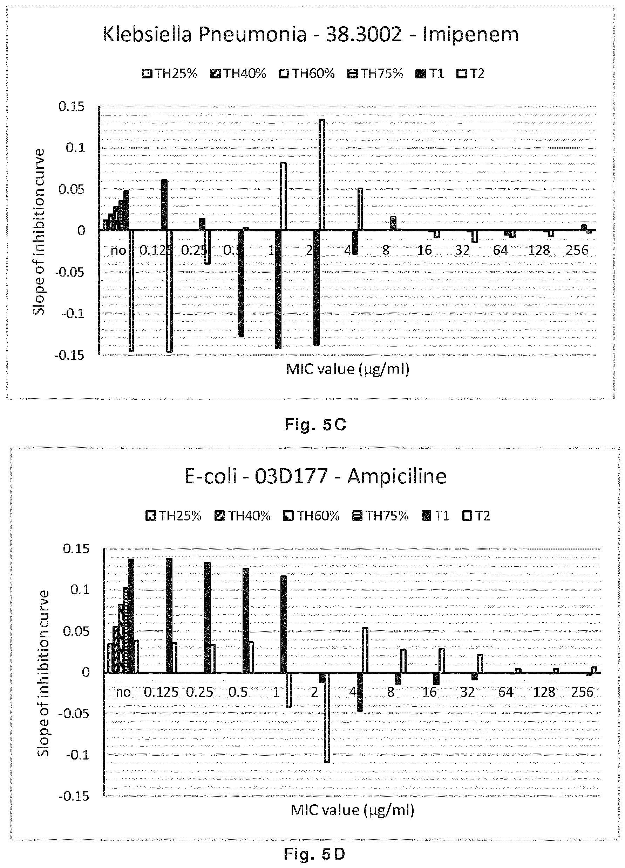

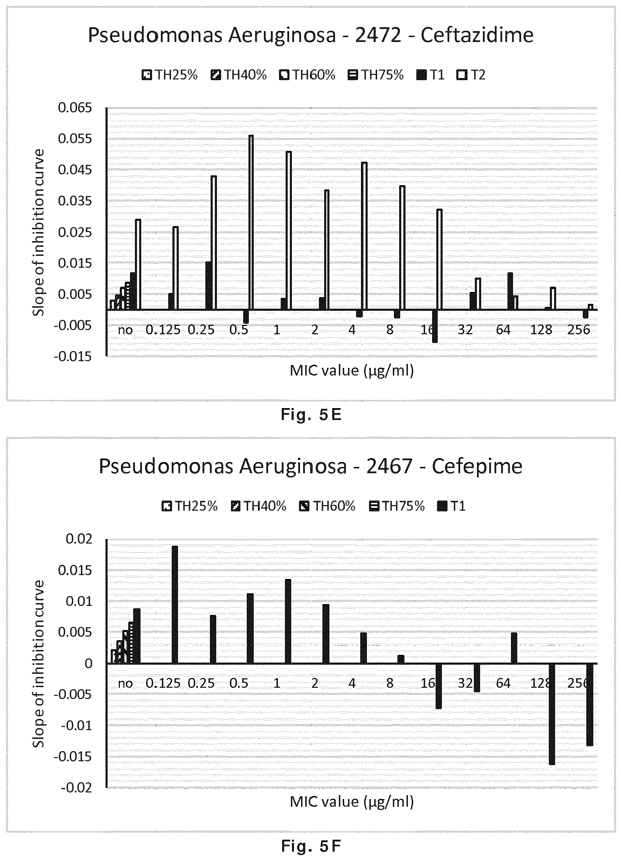

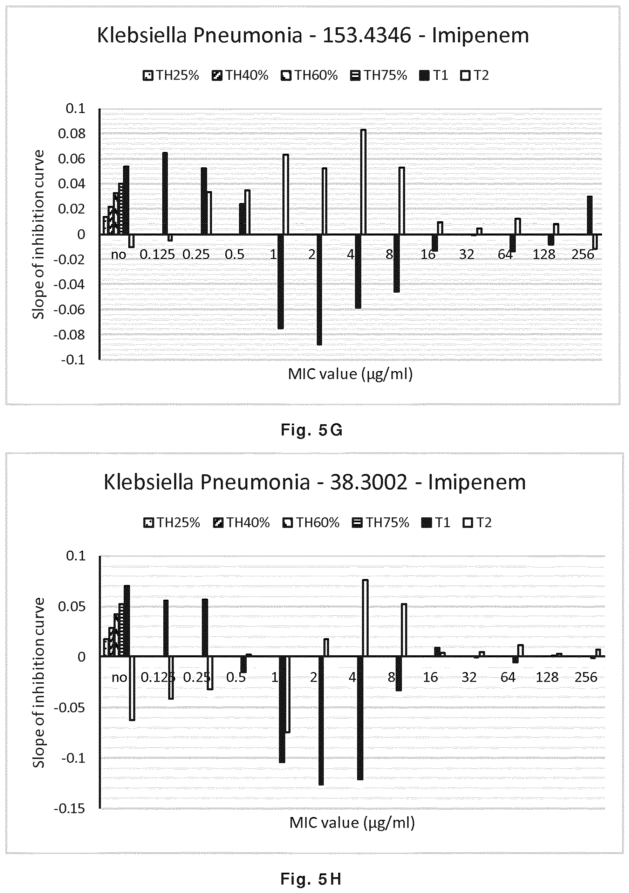

**Figures 5A, 5B, 5C, 5D, 5E, 5F, 5G and 5H** each represent a graph of the slope value of a second derivative of measured light intensities, represented by calculated absorbance values, over different antimicrobial drug concentrations in a first time frame and a second time frame and different threshold values of an experiment testing a minimum inhibitory concentration and heteroresistancy of bacteria cultures to a chemical substance in accordance with an embodiment of the disclosure.

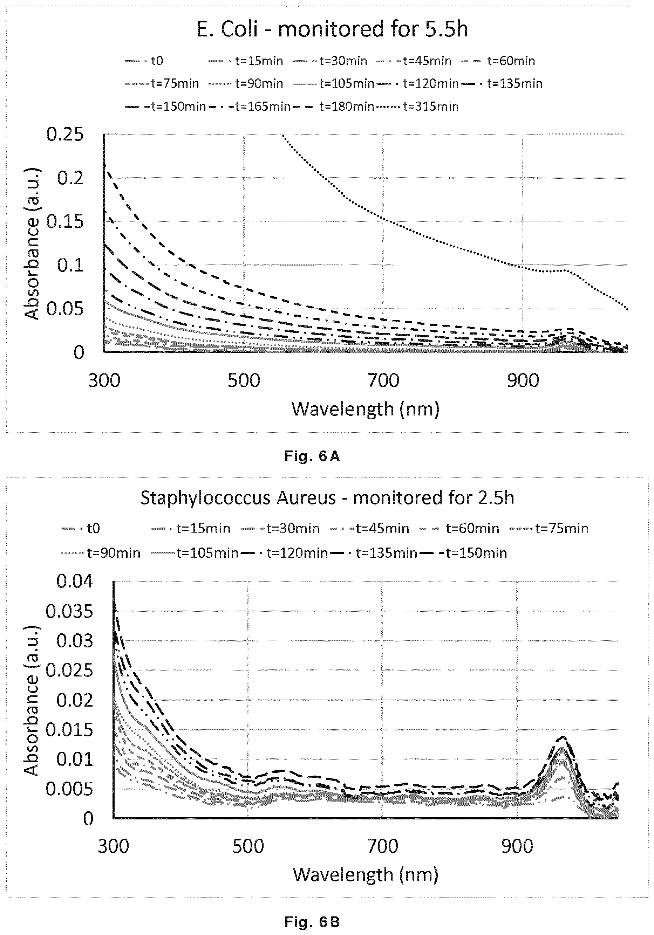

**Figure 6A and 6B** each show a bacteria culture growth measured by illuminating the bacteria culture with different wavelengths at different points in time and measuring the transmitted light intensity, whereby the absorbance, representing the measured transmitted light intensity versus the incident light intensity, is plotted over the wavelength for each time point, and whereby the bacteria culture is grown in a broth.

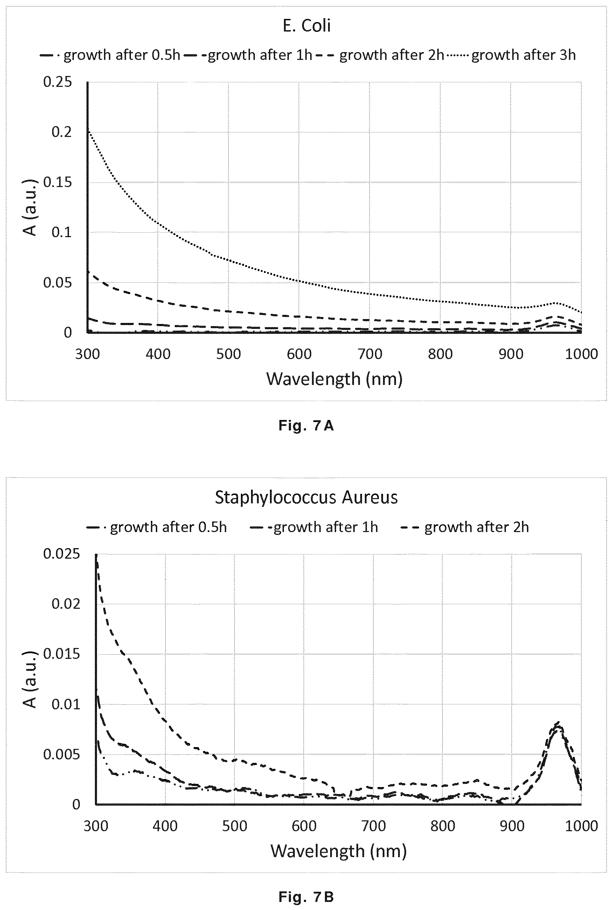

**Figure 7A and 7B** each show a bacteria culture growth measured by illuminating the bacteria culture with different wavelengths at different points in time and measuring the transmitted light intensity, whereby the absorbance, representing the measured transmitted light intensity versus the incident light intensity, is plotted over the wavelength for each time point, and whereby the bacteria culture is grown in a broth medium.

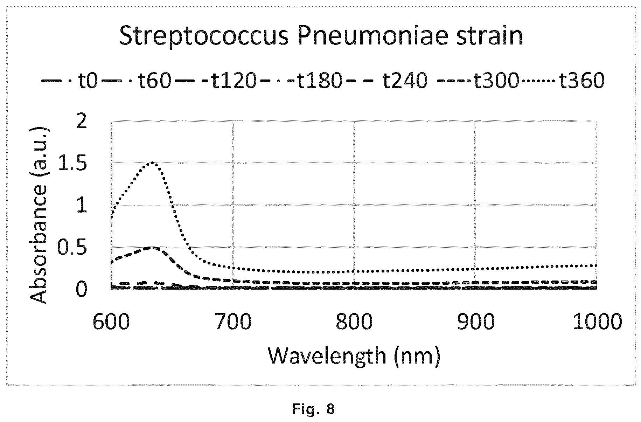

**Figure 8** shows a bacteria culture growth measured by illuminating the bacteria culture with different wavelengths at different points in time and measuring the transmitted light intensity, whereby the absorbance, representing the measured transmitted light intensity, is plotted over the wavelength for each time point.

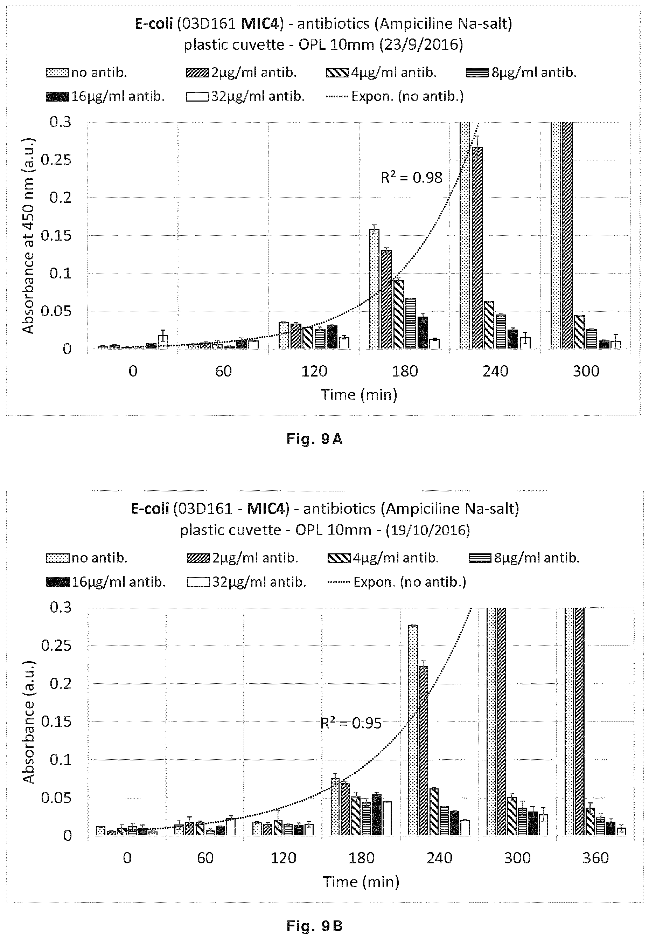

**Figure 9A and 9B** each show a bacteria culture growth in the presence of different microbial drug concentrations measured by illuminating the bacteria culture with a wavelength at different points in time and measuring the transmitted light intensity, whereby the absorbance, represented by the measured light intensity versus the incident light intensity, is plotted over time for each antimicrobial drug concentration.

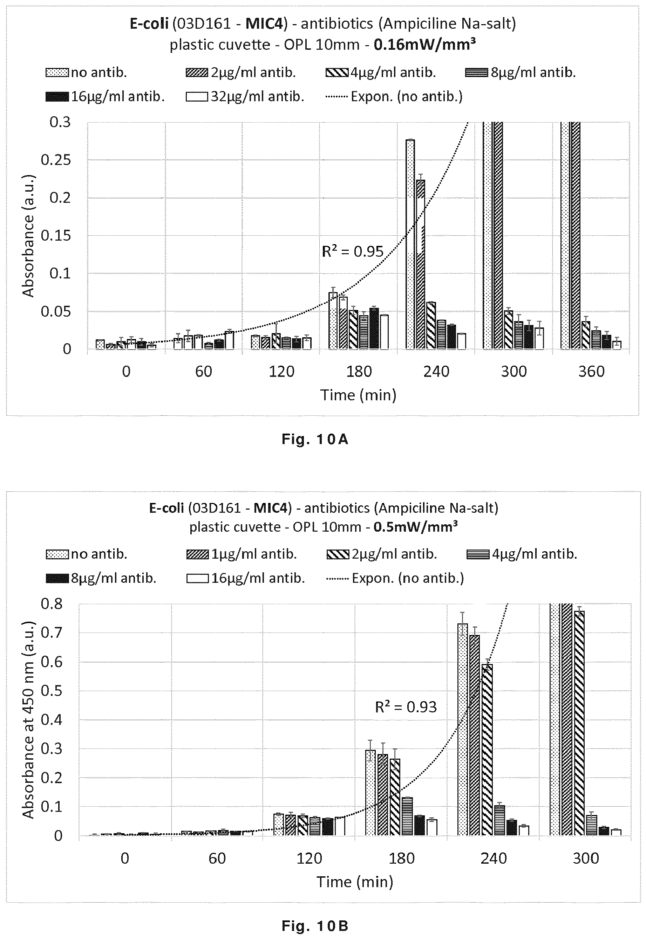

**Figure 10A and 10B** each show a bacteria culture growth in the presence of different microbial drug concentrations measured by illuminating with a illumination power the bacteria culture with a wavelength at different points in time and measuring the transmitted light intensity, whereby the absorbance, represented by the measured light intensity versus the incident light intensity, is plotted over time for each antimicrobial drug concentration.

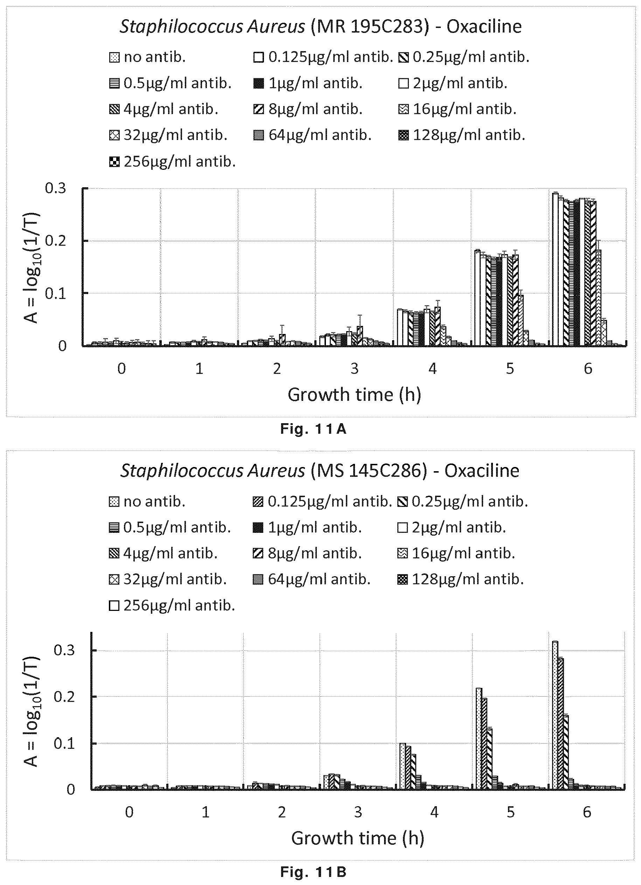

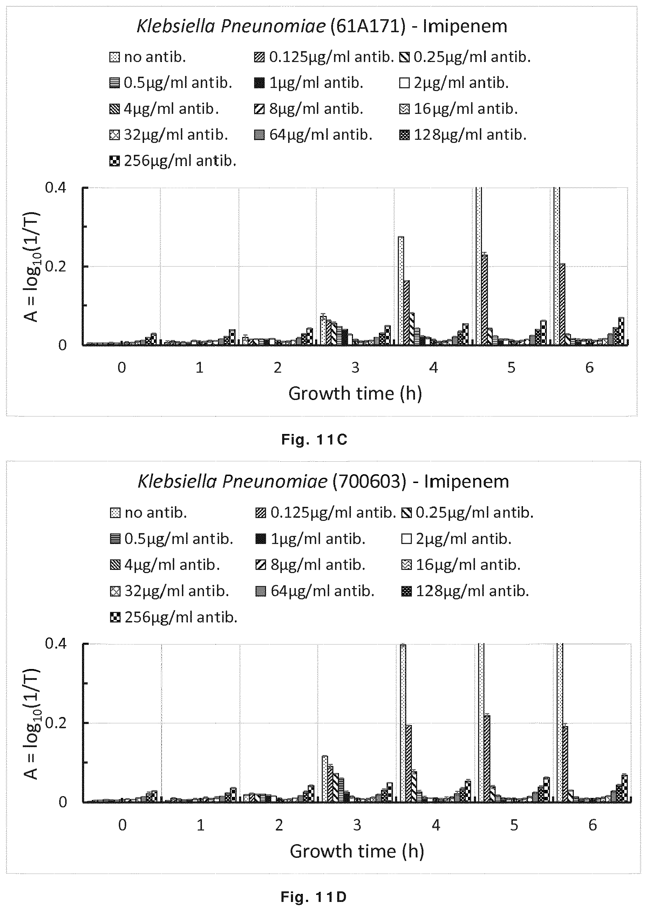

**Figure 11A, 11B, 11C and 11D** each show a bacteria culture growth in the presence of different microbial drug concentrations measured by illuminating the bacteria culture with a wavelength at different points in time and measuring the transmitted light intensity, whereby the absorbance, represented by the measured light intensity versus the incident light intensity, is plotted over time for each antimicrobial drug concentration.

**DETAILED DESCRIPTION OF THE INVENTION**

The present invention concerns a method for determining a minimum inhibitory concentration (MIC) to a microbial drug for a bacteria culture. The method also concerns determining if said bacteria culture is heteroresistant for said chemical substance.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

"Liquid growth medium" as used herein refers to any liquid composition suitable for growing a bacteria culture, e.g. blood or broth, wherein blood medium may or may not comprise a mixture of blood and broth. The liquid growth medium can be an enrichment culture medium, a selective culture medium, a differential culture medium or a resuscitation culture medium.

"Container" as used herein refers to any tool or plate comprising at least one well. A chip can refer to a cuvette, a microplate, a fluidic chip or a microfluidic chip. A chip can be a tool comprising at least one well wherein the well is not in direct contact to the atmosphere.

"Antimicrobial drug" as used herein refers to any agent that kills microorganisms (e.g. bacteria or fungi) or stops their growth. It may or may not refer to antibiotics or antifungals.

"Interaction volume" as used herein refers to a volume, whereby the volume interacts with a light source (e.g. with a laser beam or any other kind of light beam). Interaction volume may or may not alternatively refer to the surface of an illumination spot multiplied by the optical path length of the light through a liquid volume.

"Intensity of the transmitted light" as used herein may or may not also be interpreted as absorbance, whereby preferably the absorbance is determined through the Lambert-Beer law. In general, the person skilled in the art will appreciate the close correlation of a detected/measured intensity of light traversing a medium and the absorbance for the medium that can be deduced from such a detected intensity.

The present invention concerns a method for determining a minimum inhibitory concentration (MIC) to an antimicrobial drug for a bacteria culture. Preferably, the method utilizes a photodetector system comprising at least one light source and at least one photodetector. Preferably, the photodetector is adapted to provide a signal corresponding to the sensed light intensity. Preferably, the method comprises placing a container in the photodetector system, whereby said container comprises a plurality of light-transmissive wells, each well comprising a different known concentration of said antimicrobial drug and each well being provided with a substantially equal concentration and volume of said bacteria culture. Preferably, said bacteria culture comprises a bacteria concentration in a liquid growth medium. Preferably, the method further comprises periodically illuminating each well with said light source and detecting the intensity of the transmitted light with said photodetector. Preferably, at least two wells, more preferably all the wells are illuminated simultaneously, although entirely separately illuminating the wells can also be possible, though less efficient. Preferably, the method further comprises calculating for each well a third derivative of said detected intensity in a first time frame and in a second time frame, said second time frame being subsequent, preferably directly subsequent, to said first time frame. Preferably, the method further comprises comparing said third derivative of the detected intensity in both said time frames to one or more threshold values. Preferably, the method further comprises determining a first MIC value based on said comparison of said third derivative of said detected intensity in said first time frame to said one or more threshold values, and determining a second MIC value based on said comparison of said third derivative of said detected intensity in said second time frame to said one or more threshold values. Preferably, the method further comprises determining from said first and said second MIC values the MIC value of said bacteria culture.

The method allows for fast and accurate determination of the minimum inhibitory concentration of a bacteria culture to an antimicrobial drug. Especially due to the employment of the third derivative of said detected intensity in the determination of the MIC value, faster, more accurate and reproducible results can be achieved. Moreover, the method is relatively simple to employ. Only one variable needs to be measured in order to accurately determine the MIC value of the bacteria culture, without the need of employing any additional components such as tags or indicators. Further, in comparison with the currently used conventional methods (such as the broth microdilution method and the E-test), the method according to the present invention gives back a clear answer on the MIC value, which does not depend on the interpretation of the person reading the results of the test, and thereby any possible confusion regarding the determination of the correct MIC value is avoided. Hereby, the MIC value of a malicious bacteria culture present in a person or an animal can be consistently and accurately determined, resulting in an optimized treatment. Optimized treatment refers to the antibiotic concentration which would suffice in order to treat the malicious bacteria culture, avoiding the further development of multi-resistant bacteria cultures by supplying a redundantly high antibiotic concentration to the bacteria culture. Moreover, due to the efficiency of the method, the bacteria culture susceptibility to different kinds of antimicrobial drugs can be tested. The benefit therein lays in the identification of the most efficient antimicrobial drug. By identifying the most efficient drug, a treatment with a wide antimicrobial drug spectrum can be avoided, reducing the chance of creating a multi-resistant bacterial culture.

Furthermore, the person performing the test and reading the results does not need to be a skilled person in the art. The previous eliminates the need for specialized clinics for determining the MIC value of a bacteria culture. The advantage herein lays in the faster time-to-results and in the reduction of the total cost for the determination of the MIC value. Faster time-to-results is desired mainly for two different reasons. It limits the chance that a life threatening or an extremely malicious bacteria (or fungus) is spread and, the faster the time to results, the better it can be confirmed which antibiotics in which concentration could be used in order to treat the patient, therefor increasing the chance of saving the patient's life. This is extremely important as bacteria or fungi cultures are known to be able to mutate quickly, especially the fast growing cultures, obtaining possibly a resistance to the used microbial drug. Therefor, the used treatment on one patient might not be effective on the other patient, addressing the need to conduct a bacteria susceptibility test to each and every single individual suffering from a malicious bacterial infection.

The method also allows for the production of an automated and/or miniaturized setup, being either a portable device or a kit for the testing of the MIC value of a bacteria culture.

The method, according to the present invention is also suitable for determining the susceptibility of a fungus culture to an antimicrobial drug.

In a preferred embodiment time intervals between the illuminations of each well are at least 1 sec, preferably at least 10 sec, more preferably at least 20 sec, even more preferably at least 30 sec, even more preferably at least 40 sec, even more preferably at least 50 sec, even more preferably at least 1 min, even more preferably at least 2 min, even more preferably at least 3 min, even more preferably at least 4 min, even more preferably at least 5 min, even more preferably at least 6 min, even more preferably at least 7 min, even more preferably at least 8 min, even more preferably at least 9 min, even more preferably at least 10 min, even more preferably at least 11 min, even more preferably at least 12 min, even more preferably at least 13 min, even more preferably at least 14 min, even more preferably at least 15 min.

In a preferred embodiment time intervals between the illuminations of each well are at most 120 min, preferably at most 115 min, more preferably at most 110 min, even more preferably at most 105 min, even more preferably at most 100 min, even more preferably at most 95 min, even more preferably at most 90 min, even more preferably at most 85 min, even more preferably at most 80 min, even more preferably at most 75 min, even more preferably at most 70 min, even more preferably at most 65 min, even more preferably at most 60 min, even more preferably at most 55 min, even more preferably at most 50 min, even more preferably at most 45 min, even more preferably at most 40 min, even more preferably at most 35 min, even more preferably at most 30 min, even more preferably at most 25 min, even more preferably at most 20 min, even more preferably at most 15 min.

In a preferred embodiment time intervals between the illuminations of each well are between 1 sec and 120 min, preferably between 10 sec and 60 min, more preferably between 30 sec and 40 min, even more preferably between 1 min and 35 min, even more preferably between 1 min and 30 min, even more preferably between 1 min and 25 min, even more preferably between 1 min and 20 min, even more preferably between 1 min and 15 min, even more preferably between 2 min and 15 min, even more preferably between 3 min and 15 min, even more preferably between 4 min and 15 min, even more preferably between 5 min and 15 min.

Especially time intervals between 10 sec and 60 min between the illuminations of each well are beneficial for the determination of the MIC value of a bacteria culture.

In a preferred embodiment the light source is a laser or at least one light-emitting diode configured for generating a light beam comprising an illumination spot. Alternatively, the light source can be any light source such as a spectral broadband light source. The spectral broadband light source may or may not be used in combination with an optical bandpass filter. Preferably, the illumination spot shape is circular. The illumination spot shape can also be elliptical. Preferably, the illumination spot diameter is between 1 $\mu$m and 5 cm, more preferably between 100 $\mu$m and 2.5 cm, even more preferably between 300 $\mu$m and 1 cm, even more preferably between 600 $\mu$m and 5 mm, even more preferably the illumination spot diameter is 1 mm. Preferably, the surface of the illumination spot is between 0.001 mm$^2$ and 100 mm$^2$, more preferably between 0.005 mm$^2$ and 90 mm$^2$, even more preferably between 0.01 mm$^2$ and 80 mm$^2$, even more preferably between 0.02 mm$^2$ and 70 mm$^2$, even more preferably between 0.04 mm$^2$ and 60 mm$^2$, even more preferably between 0.06 mm$^2$ and 50 mm$^2$, even more preferably between 0.08 mm$^2$ and 40 mm$^2$, even more preferably between 0.1 mm$^2$ and 30 mm$^2$, even more preferably between 0.15 mm$^2$ and 20 mm$^2$, even more preferably between 0.2 mm$^2$ and 10 mm$^2$, even more preferably between 0.25 mm$^2$ and 9 mm$^2$, even more preferably between 0.3 mm$^2$ and 8 mm$^2$, even more preferably between 0.35 mm$^2$ and 7 mm$^2$, even more preferably between 0.4 mm$^2$ and 6 mm$^2$, even more preferably between 0.45 mm$^2$ and 5 mm$^2$, even more preferably between 0.5 mm$^2$ and 4 mm$^2$, even more preferably between 0.55 mm$^2$ and 3 mm$^2$, even more preferably between 0.6 mm$^2$ and 2 mm$^2$, even more preferably between 0.6 mm$^2$ and 1 mm$^2$, even more preferably between 0.6 mm$^2$ and 0.9 mm$^2$, even more preferably between 0.65 mm$^2$ and 0.85 mm$^2$, even more preferably between 0.7 mm$^2$ and 0.8 mm$^2$. Preferably, the well comprises a side wall, through which at least a part of the liquid volume is illuminated. Preferably, the well comprises a side wall, through which at least a part of the liquid volume is illuminated. Preferably, the surface of the illumination spot may or may not be equal to or smaller than the surface of the side wall. Hereby, the illumination spot dimensions are matched to the well dimensions and still allow for some alignment tolerances.

In a preferred embodiment the illumination light is collimated. This eliminates in some cases the need to use additional optical components (such as an additional light capturing lens between the container and the optical detector), allowing for a more simplified setup up.

In a preferred embodiment the light-transmissive wells are illuminated with an illumination power density of between 0.01 mW/mm$^3$ and 10.0 mW/mm$^3$, but preferably at most 5.0 mW/mm$^3$. "Illumination power density" as used herein refers to the measured illumination power at the well position divided by the interaction volume. More preferably, the illumination power density is between 0.05 mW/mm$^3$ and 1 mW/mm$^3$, even more preferably between 0.08 mW/mm$^3$ and 0.8 mW/mm$^3$, even more preferably the illumination power density is 0.5 mW/mm$^3$.

In a preferred embodiment the method further comprises determining if said third derivative of said detected intensity in said first time frame is larger than said third derivative of said detected intensity in said second time frame; if the third derivative of said detected intensity in said first time frame is smaller than the third derivative of said detected intensity in said second time frame, a new first and a new second time frame is selected, whereby said new first time frame is the subsequent time frame to said first time frame, and preferably the previous second time frame, and said new second time frame is the subsequent time frame to said second time frame; whereby the steps of determining if the third derivative of said detected intensity in the first time frame is larger than the third derivative of said detected intensity in the second time frame and selecting a new first and second time frame if the third derivative of said detected intensity in the first time frame is smaller than the third derivative of said detected intensity in the second time frame are repeated until the third derivative of said detected intensity in a new first time frame is larger than the third derivative of said detected intensity in a new second time frame, wherein said new first and second time frames are subsequently set as the first and second time frames.

An advantage can be found in the duration of the measuring time, and thus the time-to-results, and also in the optimization of the method. Utilizing the mentioned steps, will make sure than the test does not run longer than necessary. Hereby, especially for fast growing bacteria, it is possible to already determine the MIC value after 2 h. Gram-negative bacteria are mostly associated with fast growing bacteria. An example is the bacteria Escherichia coli. Escherichia coli can have a generation time of less than 10 min. However, certain Gram-positive bacteria can also be classified as fast growing. A known example is Clostridium perfringens, which can also have a regeneration time of less than 10 min. For slow growing bacteria cultures the method will make sure that the test is not stopped before a result about the MIC value of the bacteria culture can be achieved. This is achieved by prolonging the duration of the test each time the mentioned criterium is not reached.

In a preferred embodiment the method comprises calculating a second derivative of said detected intensity in said first time frame and/or said second time frame, determining if there is an inflection point the second derivative of said detected intensity in the first and/or said second time frame; whereby, if said inflection point is not present a new first and a new second time frame is selected, whereby said new first time frame is the subsequent time frame to said first time frame, and preferably the previous second time frame, and said new second time frame is the subsequent time frame to said second time frame.

By already looking at the second derivative before the third derivative is evaluated, whereby the method looks for an inflection point in the second derivative, a decision to prolong the test can be made faster and the MIC value can be determined with higher accuracy. This can be especially advantageous when the time intervals, whereby each well is periodically illuminated, are short (e.g. few minutes).

In a preferred embodiment the heteroresistancy of said bacteria culture is determined based on said first and said second MIC values, wherein said heteroresistance is determined by dividing said second and said first MIC values, whereby said bacteria culture is determined to be heteroresistant if said division gives a value greater than or equal to a first heteroresistancy threshold value. The applicant has noticed that the mentioned step increases the accuracy and reproducibility of the determination of the heteroresistancy of the bacteria culture, whereby the heteroresistancy determination is not dependent on the person reading and interpreting the outcome of the test. Therapeutic dosing of antibiotics without considering the highly resistant subpopulations of a heteroresistant culture will select for the more resistant subpopulations. This is particularly the case when the majority of the population is sensitive to antibiotics and only a small subset displays resistance above the clinical breakpoint. In these situations, antibiotic therapy will lead to eradication of the more sensitive members of the bacterial population and their replacement by the more resistant cells. Therefore the determination if a culture is heteroresistant is an important aspect when testing the susceptibility of the culture.

In a preferred embodiment the heteroresistancy of said bacteria culture is determined based on said first and said second MIC values, wherein said heteroresistance is determined by dividing said second and said first MIC values, whereby said bacteria culture is determined not to be heteroresistant if said division gives a value smaller than or equal to a second heteroresistancy threshold value. As it is important to determine if a bacteria culture is heteroresistant, it is equally important to determine with certainty that a bacteria culture is not heteroresistant.

In a preferred embodiment the heteroresistance of the bacteria culture is determined by dividing said second and said first MIC values for each threshold value, whereby the bacteria culture for a threshold value is determined to be heteroresistant if said division gives a value greater than or equal to a first heteroresistancy threshold value; whereby the bacteria culture is determined to be heteroresistant if for at least half of the threshold values, the bacteria culture is determined to be heteroresistant for the majority of the threshold values.

The applicant has noticed that by first testing the heteroresistancy for different threshold values, and based on the obtained heteroresistancy information for each threshold value determining the general heteroresistancy, the general heteroresistancy of the bacteria culture can be determined with a significantly higher accuracy and reproducibility.

In a preferred embodiment the heteroresistance of the bacteria culture is determined by dividing said second and said first MIC values for each threshold value, whereby the bacteria culture for a threshold value is determined to be heteroresistant if said division gives a value smaller than or equal to a second heteroresistancy threshold value; whereby the bacteria culture is determined to be heteroresistant if for at least half of the threshold values, the bacteria culture is determined to be heteroresistant for the majority of the threshold values.

In a preferred embodiment the first heteroresistancy threshold value is at least 1, more preferably at least 2, even more preferably at least 3, even more preferably at least 4, even more preferably at least 5, even more preferably at least 6, even more preferably at least 7, even more preferably at least 8. Preferably, the first heteroresistancy threshold value is at most 30, preferably at most 25, more preferably at most 20, even more preferably at most 15, even more preferably at most 10. Preferably, the first heteroresistancy threshold value is between 4 and 12, more preferably between 5 and 11, even more preferably between 6 and 10, even more preferably between 7 and 9, even more preferably the first heteroresistancy threshold value is 8. The first heteroresistancy threshold value can therefor be 4, 5, 6, 7, 8, 9, 10, 11, 12 or any decimal number in between 4 and 12. The applicant has noticed that a first heteroresistancy threshold value between 4 and 12, and especially a first heteroresistancy threshold of 8, allows for an accurate determination if the culture is heteroresistant.

In a preferred embodiment the second heteroresistancy threshold value is at most 11, more preferably at most 10, even more preferably at most 9, even more preferably at most 8, even more preferably at most 7, even more preferably at most 6, even more preferably at most 5, even more preferably at most 4. Preferably, the second heteroresistancy threshold value is between 0 and 8, more preferably between 1 and 7, even more preferably between 2 and 6, even more preferably between 3 and 5, even more preferably the second heteroresistancy threshold value is 4. The second heteroresistancy threshold value can therefor be 0, 1, 2, 3, 4, 5, 6, 7, 8 or any decimal number in between 0 and 8. The applicant has noticed that a second heteroresistancy threshold value between 0 and 8, and especially a first heteroresistancy threshold of 4, allows for an accurate determination if the culture is not heteroresistant.

In a preferred embodiment one of said wells is a reference well comprising a concentration of 0 mg/ml of said antimicrobial drug. Preferably, said one or more threshold values are calculated based on a reference intensity defined by the detected intensity in said reference well, wherein preferably said one or more threshold values are calculated based on a reference value defined by the third derivative in said first or second time frame, preferably said first time frame, of said reference intensity.

The applicant has noticed that by making the threshold values depend on the obtained results from the illumination, the accuracy and the reproducibility of the MIC value and heteroresistancy determinations are significantly improved.

In a preferred embodiment said one or more threshold values have a value of between 5 % and 95 %, preferably between 10 % and 90 %, of a reference value defined by the third derivative in said first or second time frame, preferably said first time frame, of said reference intensity. Preferably, the one or more threshold values have a value of at most 150 %, preferably at most 140 %, more preferably at most 130 %, even more preferably at most 120 %, even more preferably at most 110 %, even more preferably at most 100 %, even more preferably at most 95 %, even more preferably at most 90 %, even more preferably at most 85 %, even more preferably at most 80 %, even more preferably at most 75 %, of a reference value defined by the third derivative in said first or second time frame, preferably said first time frame, of said reference intensity. Preferably, the one or more threshold values have a value of at least 0 %, preferably at least 5 %, more preferably at least 10 %, even more preferably at least 15 % of a reference value defined by the third derivative in said first or second time frame, preferably said first time frame, of said reference intensity.

The applicant has noticed that threshold values having a value of between 10 % and 90 % of the reference value defined by the third derivative in said first or second time frame, preferably said first time frame, of said reference intensity, the accuracy and the reproducibility of the MIC value and heteroresistancy determinations are significantly improved.

In a preferred embodiment said one or more threshold values comprise at least three threshold values. Preferably each threshold value has a different value. Preferably, a first threshold value has a value of at least 15 %, preferably at least 16 %, more preferably at least 17 %, even more preferably at least 18 %, even more preferably at least 19 %, even more preferably at least 20 %, even more preferably at least 21 %, even more preferably at least 22 %, even more preferably at least 23 %, even more preferably at least 24 %, even more preferably at least 25 %, even more preferably at least 26 %, even more preferably at least 27 %, even more preferably at least 28 %, even more preferably at least 29 %, even more preferably at least 30 %, even more preferably at least 31 %, even more preferably at least 32 %, even more preferably at least 33 %, even more preferably at least 34 %, even more preferably at least 35 %, even more preferably at least 36 %, even more preferably at least 37 %, even more preferably at least 38 %, even more preferably at least 39 %, even more preferably at least 40 %, of the reference value. Preferably, a first threshold value has a value of at most 65 %, preferably at most 64 %, more preferably at most 63 %, even more preferably at most 62 %, even more preferably at most 61 %, even more preferably at most 60 %, even more preferably at most 59 %, even more preferably at most 58 %, even more preferably at most 57 %, even more preferably at most 56 %, even more preferably at most 55 %, even more preferably at most 54 %, even more preferably at most 53 %, even more preferably at most 52 %, even more preferably at most 51 %, even more preferably at most 50 %, even more preferably at most 49 %, even more preferably at most 48 %, even more preferably at most 47 %, even more preferably at most 46 %, even more preferably at most 45 %, even more preferably at most 44 %, even more preferably at most 43 %, even more preferably at most 42 %, even more preferably at most 41 %, even more preferably at most 40 %, of the reference value. Preferably, a second threshold value has a value of at least 45 %, preferably at least 46 %, more preferably at least 47 %, even more preferably at least 48 %, even more preferably at least 49 %, even more preferably at least 50 %, even more preferably at least 51 %, even more preferably at least 52 %, even more preferably at least 53 %, even more preferably at least 54 %, even more preferably at least 55 %, even more preferably at least 56 %, even more preferably at least 57 %, even more preferably at least 58 %, even more preferably at least 59 %, even more preferably at least 60 %, of the reference value. Preferably, the second threshold value has a value of at most 75 %, preferably at most 74 %, more preferably at most 73 %, even more preferably at most 72 %, even more preferably at most 71 %, even more preferably at most 70 %, even more preferably at most 69 %, even more preferably at most 68 %, even more preferably at most 67 %, even more preferably at most 66 %, even more preferably at most 65 %, even more preferably at most 64 %, even more preferably at most 63 %, even more preferably at most 62 %, even more preferably at most 61 %, even more preferably at most 60 %, of the reference value. Preferably, a third threshold value has a value of at least 60 %, preferably at least 61 %, more preferably at least 62 %, even more preferably at least 63 %, even more preferably at least 64 %, even more preferably at least 65 %, even more preferably at least 66 %, even more preferably at least 67 %, even more preferably at least 68 %, even more preferably at least 69 %, even more preferably at least 70 %, even more preferably at least 71 %, even more preferably at least 72 %, even more preferably at least 73 %, even more preferably at least 74 %, even more preferably at least 75 %, of the reference value. Preferably, the third threshold value has a value of at most 90 %, preferably at most 89 %, more preferably at most 88 %, even more preferably at most 87 %, even more preferably at most 86 %, even more preferably at most 85 %, even more preferably at most 84 %, even more preferably at most 83 %, even more preferably at most 82 %, even more preferably at most 81 %, even more preferably at most 80 %, even more preferably at most 79 %, even more preferably at most 78 %, even more preferably at most 77 %, even more preferably at most 76 %, even more preferably at most 75 %, of the reference value.

In a preferred embodiment the method is adapted for classifying said bacteria culture in two or more culture types, whereby one of said wells is a reference well comprising a concentration of 0 mg/ml of said antimicrobial drug. Preferably the method comprises calculating in said reference well a first derivative of said detected intensity in a predetermined time frame; comparing said first derivative in said predetermined time frame to a growth speed threshold value; classifying said bacteria culture in a culture type based on said comparison of said first derivative in said predetermined time frame to said growth speed threshold value; wherein said one or more threshold values depend on the classification of said bacteria in a culture type.

The applicant has noticed that by classifying the culture in a type, and slightly adapting the method according to the classified type, the accuracy and reproducibility of the MIC value and/or heteroresistancy determination is significantly improved.

In a preferred embodiment the bacteria culture is classified in one of two types. Preferably, the bacteria culture is classified either in a first type or a second type. The first type corresponds to faster growing bacteria cultures. The second type corresponds to slower growing bacteria cultures. The applicant has noticed that mainly the growth speed of the bacteria culture is important to take into account when determining the MIC value and/or the heteroresistancy of the bacteria culture.

In a preferred embodiment the method comprises at least four threshold values if the bacteria culture is classified in the second type. Preferably, the method comprises at least three threshold values if the bacteria culture is classified in the first type. This is advantageous for increasing the accuracy and reproducibility of the MIC value and/or heteroresistancy determination.

In a preferred embodiment said derivative is a numerical differentiation of said measured intensities.

In a preferred embodiment said comparison of said third derivative of said detected intensity for each well in said both time frames to said one or more threshold values, comprises determining for each well if said third derivative of said detected intensity in said first time frame is higher or lower than said one or more threshold values, and if said third derivative of said detected intensity in said second time frame is higher or lower than said one or more threshold values.

The applicant has noticed that seeing if the third derivative of the detected intensity in a time frame is higher, lower or equal to the one or more threshold values sufficed for the correct first and second MIC values determination. Further, this is mathematically not a difficult step, and if implemented into a program it would not require much computational time. Therefor being beneficial for achieving faster results.

In a preferred embodiment said determination of said first and said second MIC values comprises determining the well with the lowest concentration of the antimicrobial drug, whereby the third derivative of said detected intensity in the respective time frame of the well is lower than said one or more threshold values and whereby all other wells containing a higher concentration of said antimicrobial drug also have the third derivative of said detected intensity in the respective time frame smaller than said one or more threshold values; and selecting the antimicrobial drug concentration of said well as said first MIC value in said first time frame and the antimicrobial drug concentration of said well as said second MIC value in said second time frame. The advantage hereof lays in the accurate determination of both the MIC value and the heteroresistance of the bacteria culture. Preferably, the selection of the second MIC value is also based on if the to be selected second MIC value is higher than the corresponding first MIC value. Hereby, preferably, the second MIC value is selected by determining the well with the lowest concentration of the antimicrobial drug, whereby the third derivative of said detected intensity in the second time frame of the well is lower than said one or more threshold values, whereby all other wells containing a higher concentration of said antimicrobial drug also have the third derivative of said detected intensity in the second time frame smaller than said one or more threshold values and whereby the well with the lowest concentration of the antimicrobial drug is a well comprising a antimicrobial drug concentration larger than or equal to the determined first MIC value. The applicant has noticed that through the incorporation of the condition, a more accurate determination of the MIC value and heteroresistance is achieved.

In a preferred embodiment the determination of the MIC value depends on the presence of heteroresistancy in the bacteria culture. When the bacteria culture is determined to be heteroresistant, the MIC value is determined by finding the largest second MIC value and assigning the largest second MIC value as the MIC value. When the bacteria culture is determined not to be heteroresistant the MIC value is determined by finding the largest first MIC value and assigning the largest first MIC value as the MIC value. Through the employment of the previous, the method makes sure the right antibiotic concentration is administered, taking the heteroresistancy into account. Preferably, if the heteroresistancy could not be determined, the MIC value is determined by finding the largest first MIC value and assigning the largest first MIC value as the MIC value.

In an alternative embodiment, the MIC value of the bacteria culture may or may not depend on one predetermined threshold value.

In a preferred embodiment if said third derivative in said first time frame is still smaller that said third derivative in said second time frame and the endpoint of said second time frame is a predetermined value, said steps comprising said second time frame are not carried out, in which case the MIC value is determined based on said first time frame. Preferably, said predetermined value is at most 12 h, preferably at most 10 h, more preferably at most 8 h, preferably at most 6 h.

Through the employment of the predetermined value for the endpoint, the duration of the test to determine MIC value and/or heteroresistancy is restricted. The applicant observed that periodically measuring the transmitted light intensity for 6 h is enough to determine the MIC value for almost any bacteria. Of course, certain strains may grow particularly slow, and could warrant a longer measuring time. Nonetheless, it is the applicant's belief that the current test should handle at least 80-90% of the average testing sample in clinics in 4-5 hours, and would even exceed 95% when measuring for 6 hours.

In a preferred embodiment the method comprises a step of finding an inflection point in a second derivative of said detected intensity and selecting said first and said second time frame based on said inflection point, preferably whereby the first time frame precedes the inflection point and the second time frame succeeds the inflection point. In an alternative embodiment the first and second time frame are predetermined values. Preferably, the second time frame is directly subsequent to the first time frame. Preferably, the first time frame is the first time frame after the wells have been periodically illuminated for 2 h.

In a preferred embodiment, each well comprises a minimal interaction volume of 5.0 mm$^3$, preferably 7.5 mm$^3$, more preferably 10.0 mm$^3$, even more preferably 15.0 mm$^3$ or even 20.0 mm$^3$.

In a preferred embodiment, each well comprises a maximal interaction volume 200.0 mm$^3$, preferably 150.0 mm$^3$, more preferably 125.0 mm$^3$, even more preferably 100.0 mm$^3$, or even 80.0 mm$^3$, 70.0 mm$^3$, 60.0 mm$^3$, 50.0 mm$^3$, 40.0 mm$^3$ or 30.0 mm$^3$.

The invention is further described by the following non-limiting examples which further illustrate the invention, and are not intended to, nor should they be interpreted to, limit the scope of the invention.

**Examples**

The present invention will now be further exemplified with reference to the following example(s). The present invention is in no way limited to the given examples or to the embodiments presented in the figures.

Example 1: Method

To better exemplify reference is made to Fig. 1 and 2. Fig. 1 shows a diagram representing a method in accordance with the present invention for the determination of the type bacteria culture (if the bacteria culture is slow or fast growing) and the determination of a first and a second time frame to be used in the determination of a minimum inhibitory concentration of said bacteria culture. Fig. 2 shows a diagram representing a method in accordance with the present invention for the determination of a minimum inhibitory concentration of said bacteria culture and if said bacteria culture is heteroresistant or not. Preferably, the method represented in Fig. 2 is subsequent or parallel to the method of Fig. 1.

The methods of Fig. 1 and 2 preferably comprise the step of placing a container in a photodetector system. Preferably, said container comprises a plurality of light-transmissive wells, each well comprising a different known concentration of said antimicrobial drug and each well being provided with a substantially equal concentration and volume of said bacteria culture. Preferably, said bacteria culture comprises a bacteria concentration in a liquid growth medium. Preferably, at least one well of container is a reference well and comprises 0 mg/mL of said antimicrobial drug. Preferably, each well is periodically illuminated by a light source and the transmitted light intensity is measured by a photodetector. Multiple wells may or may not be simultaneously periodically illuminated.

A first, a second and a third derivative as used in this example refer to respectively a first, a second and a third derivative of the measured light intensity.

The method of Fig. 1 starts at a time equal to zero, t0 (1). Preferably, said t0 is the moment a pure bacteria culture is finished being prepared. Alternatively, said t0 is the moment all wells contain both said bacteria culture and said antimicrobial drug. After the starting of the experiment each well is periodically illuminated with a light source and the intensity of the transmitted light is detected with a photodetector (2). Preferably, each well is illuminated and the intensity of the transmitted light is detected every hour (2). More preferably, each well is illuminated and the intensity of the transmitted light is detected every 5 to 15 min. After a predetermined time length, preferably after 4 hours, a first, a second and a third derivative of said measured intensity is calculated (3) in a first and a second time frame for each well. Preferably, said second time frame is the last measured time frame after the test has been running for said predetermined time length, and said first time frame is the time frame preceding the second time frame. More preferably, said first time frame is between two and three hours and said second time frame is between three and four hours. After the calculation of said derivatives, said first derivative in said second time frame of said reference well is compared to a growth speed threshold value (4). Preferably, said growth speed threshold value is 0.166 (4). If said first derivative in said second time frame of said reference well is larger than or equal to said growth speed threshold value said bacteria culture is classified as Type I (4). If said first derivative in said second time frame of said reference well is smaller than said growth speed threshold value said bacteria culture is classified as Type II (4). Subsequently, independent of the classification of the bacteria culture according to the two types, the method comprises the following steps which are performed for each well or for all wells except said reference well. Both said first and said second time frames are evaluated to see if there is an inflection point present in the second derivative in said first and/or said second time frame (5). If said first and/or said second time frame comprise said inflection point than said third derivative in said first time frame is compared to said third derivative in said second time frame (6). If said third derivative in said first time frame is larger than said third derivative in said second time frame, then said first time frame and said second time frame are selected as a first and second time frame to be used in a minimal inhibitory concentration determination method (10). If said third derivative in said first time frame is not larger than said third derivative in said second time frame, then said well is illuminated again after a certain time period, preferably an hour, more preferably 5 to 15 min after said predetermined time length said, with a light source and the intensity of the transmitted light is detected with a photodetector forming a new time frame subsequent to said second time frame (7). A third derivative, and possibly also a first and/or a second derivative, is calculated in said new time frame (7). Further, in step (7) a new first time frame is put equal to said second time frame and a new second time frame to equal to said new time frame. Subsequently, said third derivative in said new first time frame is compared to said third derivative in said new second time frame (8). If said derivative in said new first time frame is larger than said third derivative in said new second time frame, then said new first time frame and said new second time frame are selected as a first and second time frame to be used in a minimal inhibitory concentration determination method (10). If said third derivative in said new first time frame is not larger than said third derivative in said new second time frame, then steps (7) and (8) are repeated again, whereby said well is illuminated again after a certain time period, preferably an hour, more preferably 5 to 15 min after the last illumination. If necessary steps (7) and (8) are repeated until a predetermined end time is reached (9), preferably said predetermined end time is six hours. If at said end time said third derivative in a new first time frame is still smaller than said third derivative in a new second time frame, then said new second time frame is selected as a first time frame to be used in a minimal inhibitory concentration determination method (10) and a second time frame is determined not to exist.

If said first or said second time frame do not comprise said inflection point than subsequently to step (5), then steps (7) and (8) are preformed after step (5) (step (6) is not preformed). If said third derivative in said first time frame is not larger than said third derivative in said, then steps (7) and (8) are repeated again, whereby said well is illuminated again after a certain time period, preferably an hour after the last illumination. If necessary steps (7) and (8) are repeated until a predetermined end time is reached (9), preferably said predetermined end time is six hours. If at said end time said third derivative in said first time frame is still smaller than said third derivative in said second time frame, then said new second time frame is selected as a first time frame to be used in a minimal inhibitory concentration determination method (10) and a second time frame is determined not to exist.

The method of Fig. 2 starts with knowledge on the type of the bacteria culture and a first and a second time frame (11). Preferably, whereby said type classifies said bacteria culture in a Type I or a Type II. Said bacteria culture can be either slower (Type II) or faster growing (Type I) culture. First, a third derivative in said first time frame of said reference well is calculated (12). Preferably, if said bacteria culture is a Type I culture, four threshold values are calculated, a first, a second, a third and a fourth threshold value (13). Preferably, said first, said second, said third and said fourth threshold values are respectively 25 %, 40 %, 60 % and 75 % of said third derivative in said first time frame of said reference well (13). Preferably, if said bacteria culture is a Type II culture, three threshold values are calculated, a first, a second and a third threshold value (14). Preferably, said first, said second and said third threshold values are respectively 40 %, 60 % and 75 % of said third derivative in said first time frame of said reference well (14). In both cases the method comprises the following steps.

For each well or for all wells except said reference well a first minimum inhibitory concentration is determined for each threshold value in said first time frame (15). Said determination of said first minimum inhibitory concentration for each threshold value is achieved by finding the well whereby the third derivative in the first time frame of the well is lower than the threshold value and whereby all other wells containing a higher concentration of said antimicrobial drug also have the third derivative in the first time frame smaller than the threshold value, and selecting the antimicrobial drug concentration of the well as said first MIC value in said first time frame, this is repeated for each threshold value (15). Preferably, if said bacteria culture is a Type I, four first minimum inhibitory concentration are determined. Preferably, if said bacteria culture is a Type II, three first minimum inhibitory concentration are determined.

If said second time frame exists, a second minimum inhibitory concentration is determined for each threshold value in said second time frame (17). Said determination of said second minimum inhibitory concentration for each threshold value is achieved by finding the well whereby the third derivative in the second time frame of the well is lower than the threshold value, whereby all other wells containing a higher concentration of said antimicrobial drug also have the third derivative in the second time frame smaller than the threshold value, and whereby the well comprises an antimicrobial concentration higher than or equal to the first minimum inhibitory concentration, and selecting the antimicrobial drug concentration of the well as said second minimum inhibitory concentration in said second time frame, this is repeated for each threshold value (17). Preferably, if said bacteria culture is a Type I, four second minimum inhibitory concentration are determined. Preferably, if said bacteria culture is a Type II, three second minimum inhibitory concentration are determined. Each second minimum inhibitory concentration is divided by the corresponding first minimum inhibitory concertation ($MIC_{T2}/MIC_{T1}$), whereby corresponding refers to the minimum inhibitory concentrations evaluated by the same threshold value. If said division is larger than or equal to a first heteroresistancy threshold value, said division is classified to be heteroresistant. If said division is smaller than or equal to a second heteroresistancy threshold value, said division is classified to not be heteroresistant. The total amount of divisions is equal to the number of threshold values (preferably, four for Type I bacteria culture and three for Type II bacteria culture). The method further comprises counting for how many threshold values said bacteria culture was determined to be heteroresistant and not heteroresistant (19). If said bacteria culture was determined to be heteroresistant more times than not heteroresistant, then said bacteria culture is determined to be heteroresistant (19). In this case the minimum inhibitory concentration of said bacteria culture is equal to the largest minimum inhibitory concentration of said second minimum inhibitory concentrations (20). If said bacteria culture was determined to be not heteroresistant more times than heteroresistant than said bacteria culture is determined not to be heteroresistant (19). In this case the minimum inhibitory concentration of said bacteria culture is equal to the largest minimum inhibitory concentration of said first minimum inhibitory concentrations (21). If said bacteria culture was determined to be heteroresistant an equal amount of times as it was determined to be not heteroresistant than no conclusion can be drawn regarding the heteroresistancy of said bacteria culture (22). In this case the minimum inhibitory concentration of said bacteria culture is equal to the largest minimum inhibitory concentration of said first minimum inhibitory concentrations (22).

If said second time frame does not exist said minimum inhibitory concentration of said bacteria culture is equal to the largest first minimum inhibitory concentration of said first minimum inhibitory concentrations, and no conclusion could be drawn on the heteroresistancy of said bacteria culture (22).

Example 2: Trial data (bacteria culture in broth) confirming the working of the method

To better exemplify reference is made to Fig. 3, 4A, 4B, 4C, 4D, 4E, 4F, 4G, 4H, 5A, 5B, 5C, 5D, 5E, 5F, 5G and 5H.

A first, a second and a third derivative as used in this example refer to respectively a first, a second and a third derivative of the measured light intensity.

Fig. 3 represents different experiments whereby different bacteria cultures where grown for 4 h without the presence of any antimicrobial substance. The results of these experiments are illustrated in the graph by plotting a first derivative of absorption values calculated from the measured light intensities over the growth time, whereby a wavelength of about 450 nm was used to illuminated the wells. The graph shows significant spacing between two groups of bacteria. The first group represents Type I or the faster growing bacterial. The second group represents Type II or the slower growing bacteria. The growth speed threshold value (0.166) is located in the significant spacing between two groups of bacteria.

Table 1 shows the numerical results of an experiment whereby a bacteria culture was tested for the determination of the minimal inhibitory concentration (MIC) and if heteroresistancy is present in the bacteria culture. Table 2 shows a summary of the conclusions, whereby the conclusion where also compared to a corresponding E-test and a corresponding broth microdilution (BMD). E-test allows for testing both the MIC value and the heteroresistancy of the bacteria culture. BMD can only determine the MIC value of the bacteria culture. Threshold 25, Threshold 40, Threshold 60 and Threshold 75 are calculated by taking respectively 25 %, 40 %, 60 % and 75 % of 0.030396 (the third derivative value in the first time frame of the well comprising 0 µg/ml antimicrobial drug). In Table 1 "no" is placed if the third derivative value is higher than a threshold value. "yes" is placed if the third derivative value is lower than a threshold value. In the first time frame (2-3 h) for each threshold value the MIC value is determined by finding the first "yes" in the row after which the row does not contain a "no" anymore, and selecting the corresponding antimicrobial drug concentration as the MIC value. In the second time frame (3-4 h) for each threshold value the MIC value is determined by finding the first "yes" in the row after which the row does not contain a "no" anymore and for which the corresponding concentration antimicrobial drug concentration is at least equal to the first MIC value of the same threshold value, the corresponding concentration is selected as the MIC value. For threshold 25, 256 is selected as the MIC value in the first time frame and also in the second time frame. In the second time frame 64 is not selected as the MIC value as 64 is smaller than 256. By taking the ratio of the MIC value in the second time frame over the MIC value in the first time frame, for each threshold, heteroresistancy can be determined. If the ratio of the MIC value in the second time frame over the MIC value in the first time frame is higher than or equal to 8 then the ratio is labelled to be heteroresistant. If the ratio of the MIC value in the second time frame over the MIC value in the first time frame is lower than or equal to 4 then the ratio is labelled not to be heteroresistant. This can be also seen in Table 2, wherein not heteroresistant is labelled with a no (which was the case for all the threshold values). For all four threshold values the ratio was labelled as no, therefor the bacteria culture is determined not to be heteroresistant and the MIC value is determined to be 256. These results are in accordance with the results obtained both from the E-test and BMD.

EP 3 767 631 A1

Table 1

| Chemical substance concentration μg/mL | 0 | 0.0125 | 0.025 | 0.05 | 0.1 | 0.2 | 0.4 | 0.8 | 1.6 | 3.2 | 64 | 128 | 256 |
|---|---|---|---|---|---|---|---|---|---|---|---|---|---|
| Third derivative value in 2-3 h | 0,030396 | 0,010110 | 0.012558 | 0.056906 | 0.029306 | 0.027295 | 0,039085 | 0.025384 | 0,013678 | 0.009315 | 0,018124 | 0,029543 | 0.004367 |
| Third derivative value in 3-4 h | -0.057484 | -0.008498 | -0.003155 | -0.069475 | -0.02295 | -0.04149 | -0.09085 | -0.02315 | 0.00434 | 0.017123 | 0.001609 | -0.04308 | -0.05141 |
| Threshold 25 | 0,007599 | "No" | "No" | "No" | "No" | "No" | "No" | "No" | "No" | "No" | "No" | "No" | "Yes" |
| | | "Yes" | "Yes" | "Yes" | "Yes" | "Yes" | "Yes" | "Yes" | "No" | "Yes" | "Yes" | "Yes" | "Yes" |
| Threshold 40 | 0.012158 | "Yes" | "No" | "No" | "No" | "No" | "No" | "No" | "No" | "Yes" | "No" | "No" | "Yes" |
| | | "Yes" | "Yes" | "Yes" | "Yes" | "Yes" | "Yes" | "Yes" | "Yes" | "No" | "Yes" | "Yes" | "Yes" |
| Threshold 60 | 0.018237 | "Yes" | "Yes" | "No" | "No" | "No" | "No" | "No" | "Yes" | "Yes" | "Yes" | "No" | "Yes" |
| | | "Yes" | "Yes" | "Yes" | "Yes" | "Yes" | "Yes" | "Yes" | "Yes" | "Yes" | "Yes" | "Yes" | "Yes" |
| Threshold 75 | 0.022797 | "Yes" | "Yes" | "No" | "No" | "No" | "No" | "No" | "Yes" | "Yes" | "Yes" | "No" | "Yes" |
| | | "Yes" | "Yes" | "Yes" | "Yes" | "Yes" | "Yes" | "Yes" | "Yes" | "Yes" | "Yes" | "Yes" | "Yes" |

*Table 2*

| AC/2435/TZ | T1 | T2 | HR? |
|---|---|---|---|
| | 2h-3h | 3h-4h | |
| E-test 256 or '>256 | | | no |
| BMD 256 or '>256 | | | |
| MIC (25%) | 256 | 256 | no |
| MIC (40%) | 256 | 256 | no |
| MIC (60%) | 256 | 256 | no |
| MIC (75%) | 256 | 256 | no |

Fig. 4A, 4B, 4C, 4D, 4E, 4F, 4G, 4H represent different experiments, whereby each graph represents a first, a second and a third derivative of measured light intensity of one well of different bacteria culture and antimicrobial drug combinations. The graphs illustrate the calculation of said derivatives. 5A, 5B, 5C, 5D, 5E, 5F, 5G and 5h represent different experiments showing the calculated threshold values and the calculated third derivative values for each well, represented by an antimicrobial drug concentration in μg/ml, in both a first time frame (T1) and a second time frame (T2).

Fig. 5A shows the results obtained for testing an Acinetobacter Species for susceptibility to imipenem. Table 3 summarizes the test results in terms of the different MIC values and heteroresistancy. As the Species is a Type II (slower growing) bacteria MIC (25%) results are omitted from participating in the determination of the MIC value and heteroresistancy of said bacteria culture. According to the method, the Acinetobacter Species is not heteroresistant and the MIC value is determined to be 0.25 μg/mL. These results are in accordance with the E-test and BMD.

*Table 3*

| AC/2390/IP | T1 | T2 | HR? |
|---|---|---|---|
| | 2h-3h | 3h-4h | |
| E-test 0.25 | | | no |
| BMD 0.25 | | | |
| MIC (25%) | 0.25 | 128 | yes |
| MIC (40%) | 0,25 | 0.25 | no |
| MIC (60%) | <0.125 | <0.125 | no |
| MIC (75%) | <0.125 | <0.125 | no |

Fig. 5B shows the results obtained for testing a Klebsiella pneumonia culture for susceptibility to imipenem. Table 4 summarizes the test results in terms of the different MIC values and heteroresistancy. As the culture is a Type I (faster growing) bacteria, MIC (25%) results are not omitted from participating in the determination of the MIC value and the heteroresistancy of said bacteria culture. According to the method, the Klebsiella pneumonia culture is heteroresistant and the MIC value is determined to be 16 μg/mL. These results are in accordance with the E-test.

Table 4

| KP/072.3489/IP | T1 | T2 | HR? |
|---|---|---|---|
| | 3h-4h | 4h-5h | |
| E-test | | | yes |
| 16 | | | |
| BMD | | | |
| 32 | | | |
| MIC (25%) | 0.5 | 16 | yes |
| MIC (40%) | 0.5 | 8 | yes |
| MIC (60%) | 0.25 | 8 | yes |
| MIC (75%) | <0.125 | 8 | yes |

Fig. 5C shows the results obtained for testing a Klebsiella pneumonia culture for susceptibility to imipenem. Table 5 summarizes the test results in terms of the different MIC values and heteroresistancy. As the culture is a Type I (faster growing) bacteria, MIC (25%) results are not omitted from participating in the determination of the MIC value and the heteroresistancy of said bacteria culture. According to the method, the Klebsiella pneumonia culture is heteroresistant and the MIC value is determined to be 16 $\mu$g/mL. These results are in accordance with the E-test and BMD.

Table 5

| KP/38-3002/Imp | T1 | T2 | HR? |
|---|---|---|---|
| | 3h-4h | 4h-5h | |
| E-test | | | yes |
| 16 | | | |
| BMD | | | |
| 16 | | | |
| MIC (25%) | 16 | 16 | no |
| MIC (40%) | 0.25 | 8 | yes |
| MIC (60%) | 0.25 | 8 | yes |
| MIC (75%) | 0.25 | 8 | yes |

Fig. 5D shows the results obtained for testing a Escherichia coli culture for susceptibility to ampicillin. Table 6 summarizes the test results in terms of the different MIC values and heteroresistancy. As the culture is a Type I (faster growing) bacteria, MIC (25%) results are not omitted from participating in the determination of the MIC value and the heteroresistancy of said bacteria culture. According to the method, the Escherichia coli culture is not heteroresistant and the MIC value is determined to be 2 $\mu$g/mL. These results are in accordance with the E-test.

Table 6

| EC/03D177/AM | T1 | T2 | HR? |
|---|---|---|---|
| | 3h-4h | 4h-5h | |
| E-test | | | no |
| 2 | | | |
| BMD | | | |
| 4 | | | |
| MIC (25%) | 2 | 8 | yes |

(continued)

| EC/03D177/AM | T1 | T2 | HR? |
|---|---|---|---|
| | 3h-4h | 4h-5h | |
| MIC (40%) | 2 | 2 | no |
| MIC (60%) | 2 | 2 | no |
| MIC (75%) | 2 | 2 | no |

Fig. 5E shows the results obtained for testing a Pseudomonas aeruginosa culture for susceptibility to ceftazidime. Table 7 summarizes the test results in terms of the different MIC values and heteroresistancy. As the Species is a Type II (slower growing) bacteria MIC (25%) results are omitted from participating in the determination of the MIC value and heteroresistancy of said bacteria culture. According to the method, the Pseudomonas aeruginosa culture is not heteroresistant and the MIC value is determined to be 32 $\mu$g/mL. These results are in accordance with the E-test and BMD.

*Table 7*

| RA/2472/CZA | T1 | T2 | HR? |
|---|---|---|---|
| | 4h-5h | 5h-6h | |
| E-test | | | no |
| 32 | | | |
| BMD | | | |
| 32 | | | |
| MIC (25%) | 64 | 64 | no |
| MIC (40%) | 32 | 32 | no |
| MIC (60%) | 32 | 32 | no |
| MIC (75%) | 32 | 32 | no |

Fig. 5F shows the results obtained for testing a Pseudomonas aeruginosa culture for susceptibility to cefepime. Table 8 summarizes the test results in terms of the different MIC values and heteroresistancy. As the Species is a Type II (slower growing) bacteria MIC (25%) results are omitted from participating in the determination of the MIC value and heteroresistancy of said bacteria culture. According to the method, the Pseudomonas aeruginosa culture MIC value is determined to be 8 $\mu$g/mL. As there is only one time frame available it is not possible to determine the heteroresistancy of the culture.

*Table 8*

| PA/2467/PM | T1 | T2 | HR? |
|---|---|---|---|
| | 5h-6h | 0 | |
| E-test | | | ? |
| Na | | | |
| BMD | | | |
| 16 | | | |
| MIC (25%) | 8 | | |
| MIC (40%) | 8 | | |
| MIC (60%) | 4 | | |
| MIC (75%) | 4 | | |

Fig. 5G shows the results obtained for testing a Klebsiella pneumonia culture for susceptibility to imipenem.

Table 9 summarizes the test results in terms of the different MIC values and heteroresistancy. As the culture is a Type I (faster growing) bacteria, MIC (25%) results are not omitted from participating in the determination of the MIC value and the heteroresistancy of said bacteria culture. According to the method, the Klebsiella pneumonia culture MIC value is determined to be higher than 256 μg/mL. Heteroresistancy could not be determined.

*Table 9*

| KP/153.4346/IP | T1 | T2 | HR? |
|---|---|---|---|
|  | 3h-4h | 4h-5h |  |
| E-test |  |  | yes |
| >32 |  |  |  |
| BMD |  |  |  |
| 16 |  |  |  |
| MIC (25%) | >256 | >256 | no |
| MIC (40%) | >256 | >256 | no |
| MIC (60%) | 0.5 | 16 | yes |
| MIC (75%) | 0.5 | 16 | yes |

Fig. 5H shows the results obtained for testing a Klebsiella pneumonia culture for susceptibility to imipenem. In order to test the repeatability the test was performed twice with the same culture, Table 10 and Table 11 summarize the test results in terms of the different MIC values and heteroresistancy for the two repetitions. As the culture is a Type I (faster growing) bacteria, MIC (25%) results are not omitted from participating in the determination of the MIC value and the heteroresistancy of said bacteria culture. According to the method, the Klebsiella pneumonia culture MIC value is heteroresistant and the MIC value is determined to be 16 μg/mL for both repetitions, proving the method has a good repeatability. Moreover, these results are in accordance with the E-test and BMD.

*Table 10*

| KP/38.5002/Imp | T1 | T2 | HR? |
|---|---|---|---|
|  | 3h-4h | 4h-5h |  |
| E-test |  |  | yes |
| 16 |  |  |  |
| BMD |  |  |  |
| 16 |  |  |  |
| MIC (25%) | 0.5 | 16 | yes |
| MIC (40%) | 0.5 | 16 | yes |
| MIC (60%) | 0.5 | 16 | yes |
| MIC (75%) | 0.5 | 8 | yes |

*Table 11*

| KP/38.3002/Imp | T1 | T2 | HR? |
|---|---|---|---|
|  | 3h-4h | 4h-5h |  |
| E-test |  |  | yes |
| 16 |  |  |  |
| BMD |  |  |  |
| 16 |  |  |  |

(continued)

| KP/38.3002/Imp | T1 | T2 | HR? |
|---|---|---|---|
| | 3h-4h | 4h-5h | |
| MIC (25%) | 16 | 16 | no |
| MIC (40%) | 0.25 | 8 | yes |
| MIC (60%) | 0.25 | 8 | yes |
| MIC (75%) | 0.25 | 8 | yes |

Table 12 and Table 13 provide an essential agreement between the method in accordance with the present invention and respectively BMD and E-test for all tested bacteria cultures. The essential agreement is good, especially taking into account that the determination of microbial susceptibility with BMD and E-test is prone to contamination and/or mistakes in the read-out of the results.

*Table 12*

| Table: differences in $\log_2$ dilutions of MIC's obtained by **the method compared to BMD (all cultures)** | | | | | | | | | | | |
|---|---|---|---|---|---|---|---|---|---|---|---|
| | | | | | | | | | | | |
| **(52)** | No. (%) of isolates showing a MIC difference (in $\log_2$ dilutions) of: | | | | | | | | | | |
| <-4 | -4 | -3 | -2 | -1 | 0 | 1 | 2 | 3 | 4 | > 4 | |
| | | 2 (3.9) | 5 (9.5) | 9 (17.3) | 22 (42.3) | 2 (3.9) | 2 (3.9) | 4 (7.8) | 3 (5.7) | 3 (5.7) | |
| Essential Agreement: 76.9% | | | | | | | | | | | |
| | | | | | | | | | | | |

*Table 13*

| Table: differences in $\log_2$ dilutions of MIC's obtained by **the method compared to E-test (all cultures)** | | | | | | | | | | | |
|---|---|---|---|---|---|---|---|---|---|---|---|
| | | | | | | | | | | | |
| **(46)** | No. (%) of isolates showing a MIC difference (in $\log_2$ dilutions) of: | | | | | | | | | | |
| < -4 | -4 | -3 | -2 | -1 | 0 | 1 | 2 | 3 | 4 | >4 | |
| | | 1 (2.2) | 3 (6.5) | 5 (10.9) | 23 (50) | 7 (15) | | 3 (6.5) | 1 (2.2) | 3 (6.5) | |
| Essential Agreement: 82.6% | | | | | | | | | | | |
| | | | | | | | | | | | |

Example 3: Trial data (bacteria culture in blood) confirming the working of the method

Tables 14 and 15 show the results of experiment whereby a bacteria culture is grown in blood instead of broth. The experiments of Table 14 were conducted by illuminating the light-transmissive wells with a light having a wavelength of 635 nm. The experiments of Table 15 were conducted by illuminating the light-transmissive wells with a light having a wavelength of 650 nm. From the test results whereby a light having a wavelength of 635 nm is used to illuminate the light-transmissive wells showed a better agreement of results with the E-test and BMD test compared to the results obtained by illuminating the light-transmissive wells with a light having a wavelength of 650 nm. The determination of the best wavelength may or may not be based on the commercial availability of the spectral line. The choice of the optimal wavelength may or may not be determined by calculating the absorbance for various wavelengths. The choice of the optimal wavelength can also be based on the combination of the comparison with the other two methods, the commercial availability and on the calculation of the absorbance for various wavelengths.

Table 14

| Broth + horse blood as medium | | | | | | | | | | | |
|---|---|---|---|---|---|---|---|---|---|---|---|
| 635 nm | | | | BMD | E-test | MICSTRIP | HR E-test | HR MICSTRIP | 4h | 5h | 6h |
| 1 | *Streptococcus Pneumoniae* | Ceftriaxone | 2532 | 0,008 | <=0.16 | 0.016 | no | ? | | | X |
| 2 | *Streptococcus Pneumoniae* | Ceftriaxone | 2543 | 0.256 | 0.125 | 0.256 | no | ? | | | X |
| 3 | *Streptococcus Pneumoniae* | Ceftriaxone | 2544 | 0.064 | 0.024 | 0.064 | no | ? | | | X |
| 4 | *Streptococcus Pneumoniae* | Ceftriaxone | ATCC 49619 | 0,064 | 0.047 | 0.032 | no | ? | | | X |
| 5 | *Streptococcus Pneumoniae* | Benzylpenicilline | 2532 | 0,016 | 0 | 0-016 | no | ? | | | X |
| 6 | *streptococcus Pneumoniae* | Benzylpenicilline | 2543 | 2 | 1.5 | 1 | no | no | | | X |
| 7 | *Streptococcus Pneumoniae* | Benzylpenicilline | 2544 | 0.256 | 0.1 | 0.256 | no | ? | | | X |
| 8 | *Streptococcus Pneumoniae* | Benzylpenicilline | ATCC 49619 | 0.256 | 0.25 | 0.256 | no | no | | | X |

20

*Table 15*

**Broth + horse Mood as medium**

| 660 nm | | | | BMD | E-test | MICSTRIP | HR E-test | HR MICSTRIP | 4h | 5h | 6h |
|---|---|---|---|---|---|---|---|---|---|---|---|
| 1 | *Streptococcus Pneumoniae* | Ceftriaxone | 2532 | 0.008 | <= 0.16 | 0.016 | no | ? | | | X |
| 2 | *Streptococcus Pneumoniae* | Ceftriaxone | 2543 | 0.256 | 0.125 | 0.256 | no | ? | | | X |
| 3 | *Streptococcus Pneumoniae* | Ceftriaxone | 2544 | 0.064 | 0-024 | 0.064 | no | ? | | | X |
| 4 | *Streptococcus Pneumoniae* | Ceftriaxone | ATCC 49619 | 0.064 | 0.047 | >16 | no | no | | X | |
| 5 | *Streptococcus Pneumoniae* | Benzylpenicilline | 2532 | 0.016 | 0 | 0.016 | no | ? | | | X |
| 6 | *Streptococcus Pneumoniae* | Benzylpenicilline | 2543 | 2 | 1.5 | 1 | no | no | | | X |
| 7 | *Streptococcus Pneumoniae* | Benzylpenicilline | 2544 | 0.256 | 0.1 | 0.256 | no | ? | | | X |
| 8 | *Streptococcus Pneumoniae* | Benzylpenicilline | ATCC 49619 | 0.256 | 0.25 | 0.256 | no | no | | | X |

Table 16 summarises the results of table 14 and provides essential agreement values for all the experiments, and Table 17 summarises the results of Table 15 and provides essential agreement values for all the experiments. For both illumination wavelengths the essential agreement is 100 % between the different methods (the method in accordance with the present invention, E-test and BMD). The previous proves at the method according to the present invention is able to accurately determine the MIC value of a bacteria culture in blood.

"Essential agreement" as used herein refers to the percentage of MIC value results that where within ± 2 doubling dilution of the MIC value determined by the reference method.

Table 16

| Broth+horse blood as medium 635 nm) | No. cultures | No antibiotics | EA (E-test-BMD (%) | EA(Our method-BMD) (%) | EA (Our method -E-test) (%) | 4h | 5h | 6h | HR (E-test) | HR (Our method) |
|---|---|---|---|---|---|---|---|---|---|---|
| Streptococcus Pneumonlae (GP) | 4 | 2 | 100 | 100 | 100 | 0 | 0 | 8 | 8(N) | 2(N) 6(?) |

*Table 17*

| Broth-horse blood as medium (650 nm) | No. cultures 4 | No antibiotics | EA (E-test-BMD) (%) | EA (Our method-BMD) (%) | E4 (Our method -E-test) (%) | 4h | 5h | 6h | HR (E-test) | HR (Our method) |
|---|---|---|---|---|---|---|---|---|---|---|
| Streptococcus *Pneumoniae* (GP) | 4 | 2 | 100 | 100 | 100 | 0 | 1 | 7 | 8(N) | 3(N) 5(?) |

Example 4: Feasibility tests for cell growth sensing via optical means in the broad spectral region

Transmittance as a function of wavelength (transmission spectrum) of a culture medium (broth) contaminated with a selected type of bacteria was measured in the wavelength region 250-1600 nm. Over a time of 5.5 hours, a spectrum was recorded every 15 minutes. In between the measurements, the cultures were stored in an incubator at 37°C.
A spectral broadband light source was used to illuminate the sample. The investigated bacteria were Escherichia coli and Staphylococcus aureus. Samples were measured using a quartz cuvette with an optical path length (OPL) of 10mm. During measurement, the cuvette was positioned in a cuvette holder. Light guidance towards and from the cuvettes takes place in optical fibres. The device contains two additional lenses respectively between the sending fibre and the cuvette and between the cuvette and the detector. The first lens concentrates the light in a small illumination spot (~1mm in diameter) while the second lens enhances light capturing. An optical spectrum analyzer was used for recording the transmission spectra T($\lambda$). For each recorded transmission spectrum T($\lambda$), the absorbance spectrum A($\lambda$) was calculated using Lambert-Beer law:

$$A(\lambda) = -\log 10 T(\lambda)$$

From FIG 6A and 6B the optical monitoring of bacteria growth is possible in the spectral region between 300 - 900 nm. From FIG 7A and 7B shorter wavelengths (300 - 480 nm) are more sensitive to cell growth. Since the wavelength cannot be too short in order not to kill the bacteria, wavelength about 450 nm is ideal for bacteria cultures in broth. From FIG 8, for bacteria cultures in blood a wavelength about 640 nm is ideal. Cell growth monitoring was possible after 1.15 - 1.30 h of growth.

Example 5: Antibiotics susceptibility testing via optical means

Transmittance as a function of wavelength of a culture medium (broth and/or blood) contaminated with a selected type of bacteria was measured with a laser setup (containing a laser as a light source). Five additional samples were prepared and measured containing a well-defined concentration of a selected antibiotics. An OPL of 10 mm was used. Over a time of 5 h, a transmittance value was recorded every hour. The MIC values of the selected bacteria and antibiotics were known. Cultures were selected such that a broad range of different MIC values was covered.
Poly(methyl methacrylate) (PMMA) cuvettes where used. PMMA cuvettes are much cheaper compared to quartz cuvettes and also have a high transmission at the wavelength chosen (450 nm for broth and 635 nm for blood).
A laser setup, using a silicon photodiode as the detector, with an interaction volume of 25.1 mm$^3$ and an illumination power density of 0.16 mW/mm$^3$ was used. The investigated bacteria was Escherichia coli and the studied antibiotics was ampicillin which was applied in the following concentrations: 1 $\mu$g/ml, 2 $\mu$g/ml, 4 $\mu$g/ml and 8 $\mu$g/ml. The tabulated MIC values for these organisms are tabulated below:

*Table 18: Tabulated MIC values of the cultures under test.*

| Organism | Colony ID. | MIC for Ampiciline |
|---|---|---|
| *E-coli* | 03D161 | 4 |
| *E-coli* | 03D177 | 2 |
| *E-coli* | 03D179 | > 32 |

From Fig. 9A and 9B, for all tested cultures, bacterial growth slows down for an antibiotics concentration close to the MIC value. The optical readout method enables antibiotics susceptibility testing.
The method was also validated using a spectral broadband light source (halogen) combined with an optical bandpass filter.

Example 6: Antibiotics susceptibility testing via optical means - enhancing contrast by increasing the illumination power density

The contrast between successive antibiotics concentrations was enhanced by increasing the illumination power density. This can be done by replacing the laser source by a more powerful one or by a better concentration of the illumination beam at the position of the sample. If needed, the laser can be replaced by other light sources such as a light-emitting diode. Hereby, the original elliptical beam of 2.51 mm$^2$ was transformed to a circular beam of 0.785 mm$^2$.

The latter was done by using two cylindrical lenses. Note that other solutions are possible including the use of other optical components such as optical fibres or prisms. Doing this implies a decrease in the interaction volume by a factor of 3.

'First' settings (laser beam directly out of laser):

- Illumination spot shape: elliptical
- Illumination spot diameter: 3.2 mm by 1 mm
- Calculated dimension of illumination spot: $(pi*3.2mm*1mm)/4 = 2.51mm^2$
- Calculated interaction volume: $2.51mm^2*10mm$ (OPL) $= 25.1mm^3$
- Measured power at sample position: 3.97mW
- Calculated illumination power density: $3.97mW/25.1mm^3 = 0.16mW/mm^3$

'Second' settings (laser beam after cylindrical lenses):

- Illumination spot shape: circular
- Illumination spot diameter: 1mm
- Calculated dimension of illumination spot: $(pi*1mm*1mm)/4 = 0.785mm^2$
- Calculated interaction volume: $0.785mm^2*10mm$ (OPL) $= 7.85mm^3$
- Measured power at sample position: 3.97mW
- Calculated illumination power density: $3.97mW/7.85mm^3 = 0.5mW/mm^3$

From Fig. 10A and 10A can be seen that by increasing the illumination power the contrast is enhanced. Fig. 11A, 11B, 11C and 11D show that also for other bacteria cultures the contrast is increased by increasing the illumination power.

## Claims

1. A method for determining a minimum inhibitory concentration (MIC) to an antimicrobial drug for a bacteria culture in a container, the method utilizing a photodetector system comprising at least one light source and at least one photodetector, said photodetector being adapted to provide a signal corresponding to the sensed light intensity, the method comprising:

   placing a container in the photodetector system, whereby said container comprises a plurality of light-transmissive wells, each well comprising a different, known concentration of said antimicrobial drug and each well being provided with a substantially equal concentration and volume of said bacteria culture, preferably whereby said bacteria culture comprises a bacteria concentration in a liquid growth medium;
   periodically illuminating each well with said light source and detecting the intensity of the transmitted light with said photodetector;

   **characterized in that** the method further comprises the steps of:

   calculating for each well a third derivative of said detected intensity in a first time frame and in a second time frame, said second time frame being subsequent, preferably directly subsequent, to said first time frame;
   comparing said third derivative of the detected intensity in both said time frames to one or more threshold values;
   determining a first MIC value based on said comparison of said third derivative in said first time frame to said one or more threshold values, and determining a second MIC value based on said comparison of said third derivative in said second time frame to said one or more threshold values; and
   determining from said first and said second MIC values the MIC value of said bacteria culture.

2. The method according to the preceding claim 1, wherein the method comprises

   determining if said third derivative in said first time frame is larger than said third derivative in said second time frame;
   if the third derivative in said first time frame is smaller than the third derivative in said second time frame, a new first and a new second time frame is selected, whereby said new first time frame is the subsequent time frame to said first time frame, and preferably the previous second time frame, and said new second time frame is the subsequent time frame to said second time frame;

whereby the steps of determining if the third derivative in the first time frame is larger than the third derivative in the second time frame and selecting a new first and second time frame if the third derivative in the first time frame is smaller than the third derivative in the second time frame are repeated until the third derivative in a new first time frame is larger than the third derivative in a new second time frame, wherein said new first and second time frames are subsequently set as the first and second time frames.

3. The method according to any of the preceding claims 1 to 2, wherein the method comprises calculating a second derivative of said detected intensity in said first time frame and/or said second time frame, determining if there is an inflection point the second derivative of said detected intensity in the first and/or said second time frame; whereby, if said inflection point is not present a new first and a new second time frame is selected, whereby said new first time frame is the subsequent time frame to said first time frame, and preferably the previous second time frame, and said new second time frame is the subsequent time frame to said second time frame.

4. The method according to any of the preceding claims 1 to 3, wherein heteroresistancy of said bacteria culture is determined based on said first and said second MIC value, wherein said heteroresistance is determined by dividing said second and said first MIC values, whereby said bacteria culture is determined to be heteroresistant if said division gives a value greater than or equal to a first heteroresistancy threshold value.

5. The method according to the any one of the preceding claims 1 to 4, wherein heteroresistance of the bacteria culture is determined by dividing said second and said first MIC values for each threshold value, whereby the bacteria culture for a threshold value is determined to be heteroresistant if said division gives a value greater than or equal to a first heteroresistancy threshold value; whereby the bacteria culture is determined to be heteroresistant if for at least half of the threshold values, the bacteria culture is determined to be heteroresistant for the majority of the threshold values.

6. The method according to any of the preceding claims 1 to 5, wherein one of said wells is a reference well comprising a concentration of 0 mg/ml of said antimicrobial drug, and that said one or more threshold values are calculated based on a reference intensity defined by the detected intensity in said reference well, wherein preferably said one or more threshold values are calculated based on a reference value defined by the third derivative in said first or second time frame, preferably said first time frame, of said reference intensity.

7. The method according to the preceding claim 5, and optionally any other of the claims, wherein said one or more threshold values have a value of between 5 % and 95 %, preferably between 10 % and 90 %, of a reference value defined by the third derivative in said first or second time frame, preferably said first time frame, of said reference intensity.

8. The method according to any of the preceding claims 1 to 7, wherein said one or more threshold values comprise at least three threshold values, whereby each threshold value has a different value.

9. The method according to any of the preceding claims 1 to 8, wherein said one or more threshold values comprise a first threshold value having a value between about 20% and 50 %, preferably between about 30 % and 45 %, of the reference value; a second threshold value having a value between about 50 % and 80 %, preferably between about 55 % and 70 %, of the reference value; a third threshold value having a value between about 70 % and 95 %, preferably between about 75 % and 90 %.

10. The method according to any of the preceding claims 1 to 9, wherein the method is adapted for classifying said bacteria culture in two or more culture types, and whereby one of said wells is a reference well comprising a concentration of 0 mg/ml of said antimicrobial drug, the method comprising:

calculating in said reference well a first derivative of said detected intensity in a predetermined time frame; comparing said first derivative in said predetermined time frame to a growth speed threshold value; classifying said bacteria culture in a culture type based on said comparison of said first derivative in said predetermined time frame to said growth speed threshold value;

wherein said one or more threshold values depend on the classification of said bacteria in a culture type.

11. The method according to any of the preceding claims 1 to 10, wherein said derivative is a numerical differentiation of said measured intensities.

**12.** The method according to any of the preceding claims 1 to 11, wherein said comparison of said third derivative for each well in said both time frames to said one or more threshold values, comprises determining for each well if said third derivative in said first time frame is higher or lower than said one or more threshold values, and if said third derivative in said second time frame is higher or lower than said one or more threshold values.

**13.** The method according to any of the preceding claims 1 to 12, wherein said determination of said first and said second MIC values comprises determining the well with the lowest concentration of the antimicrobial drug, whereby the third derivative in the respective time frame of the well is lower than said one or more threshold values and whereby all other wells containing a higher concentration of said antimicrobial drug also have the third derivative in the respective time frame smaller than said one or more threshold values; and selecting the antimicrobial drug concentration of said well as said first MIC value in said first time frame and the antimicrobial drug concentration of said well as said second MIC value in said second time frame.

**14.** The method according to the preceding claim 2, and optionally any other claim, wherein if said third derivative in said first time frame is still smaller that said third derivative in said second time frame and the endpoint of said second time frame is a predetermined value, said steps comprising said second time frame are not carried out, in which case the MIC value is determined based on said first time frame.

**15.** A method for determining a minimum inhibitory concentration (MIC) to an antimicrobial drug for a bacteria culture in a container, the method utilizing a photodetector system comprising at least one light source and at least one photodetector, said photodetector being adapted to provide a signal corresponding to the sensed light intensity, the method comprising:

placing a container in the photodetector system, whereby said container comprises a plurality of light-transmissive wells, each well comprising a different, known concentration of said antimicrobial drug and each well being provided with a substantially equal concentration and volume of said bacteria culture, preferably whereby said bacteria culture comprises a bacteria concentration in a liquid growth medium;
periodically illuminating each well with said light source and detecting the intensity of the transmitted light with said photodetector;

**characterized in that** the method further comprises the steps of:

calculating for each well a third derivative of said detected intensity in a first time frame and in a second time frame, said second time frame being subsequent, preferably directly subsequent, to said first time frame;
comparing said third derivative of the detected intensity in both said time frames to one or more threshold values;
determining a first MIC value based on said comparison of said third derivative in said first time frame to said one or more threshold values, and determining a second MIC value based on said comparison of said third derivative in said second time frame to said one or more threshold values;
determining from said first and said second MIC values the MIC value of said bacteria culture;
determining if said third derivative in said first time frame is larger than said third derivative in said second time frame;
if the third derivative in said first time frame is smaller than the third derivative in said second time frame, a new first and a new second time frame is selected, whereby said new first time frame is the subsequent time frame to said first time frame, and preferably the previous second time frame, and said new second time frame is the subsequent time frame to said second time frame;

whereby the steps of determining if the third derivative in the first time frame is larger than the third derivative in the second time frame and selecting a new first and second time frame if the third derivative in the first time frame is smaller than the third derivative in the second time frame are repeated until the third derivative in a new first time frame is larger than the third derivative in a new second time frame, wherein said new first and second time frames are subsequently set as the first and second time frames; and wherein heteroresistancy of said bacteria culture is determined based on said first and said second MIC value, wherein said heteroresistance is determined by dividing said second and said first MIC values, whereby said bacteria culture is determined to be heteroresistant if said division gives a value greater than or equal to a first heteroresistancy threshold value.

**Fig. 1**

Fig. 2

Fig. 3

Acetinobacter Species - 2390 - Imipenem

Fig. 4 A

Klebsiella Pneumonia - 072.3489 - Imipenem

Fig. 4 B

**Klebsiella Pneumonia - 38.3002 - Imipenem**

Legend: —□— Growth rate (dA/dt)   —▲— Growth rate speed ($d^2A/dt^2$)   --◄>-- Slope

Growth time (h)

**Fig. 4 C**

**E-coli - 03D177 - Ampiciline**

Legend: —□— Growth rate (dA/dt)   —▲— Growth rate speed ($d^2A/dt^2$)   --◄>-- Slope

Growth time (h)

**Fig. 4 D**

## Pseudomonas Aeruginosa - 2472 - Ceftazidime

—□— Growth rate (dA/dt)  —▲— Growth rate speed ($d^2A/dt^2$)  —◀>— Slope

Growth time (h)

**Fig. 4 E**

## Pseudomonas Aeruginosa - 2467 - Cefepime

—□— Growth rate (dA/dt)  —▲— Growth rate speed ($d^2A/dt^2$)  —◀>— Slope

Growth time (h)

**Fig. 4 F**

**Klebsiella Pneumonia - 153.4346 - Imipenem**

—□— Growth rate (dA/dt)    —▲— Growth rate speed (d²A/dt²)    ╍►╍ Slope

Growth time (h)

Fig. 4 G

**Klebsiella Pneumonia - 38.3002 - Imipenem**

—□— Growth rate (dA/dt)    —▲— Growth rate speed (d²A/dt²)    ╍►╍ Slope

Growth time (h)

Fig. 4 H

Fig. 5 A

Fig. 5 B

Fig. 5C

Fig. 5D

Fig. 5 E

Fig. 5 F

Fig. 5G

Fig. 5H

Fig. 6 A

Fig. 6 B

Fig. 7 A

Fig. 7 B

Fig. 8

**Fig. 9 A**

**Fig. 9 B**

Fig. 10A

Fig. 10B

## *Staphilococcus Aureus* (MR 195C283) - Oxaciline

⊞ no antib.      □ 0.125µg/ml antib. ◨ 0.25µg/ml antib.
▤ 0.5µg/ml antib.  ■ 1µg/ml antib.     □ 2µg/ml antib.
▨ 4µg/ml antib.   ▨ 8µg/ml antib.     ⊠ 16µg/ml antib.
⊠ 32µg/ml antib.  ▨ 64µg/ml antib.    ▦ 128µg/ml antib.
▨ 256µg/ml antib.

**Fig. 11A**

## *Staphilococcus Aureus* (MS 145C286) - Oxaciline

⊞ no antib.      ▨ 0.125µg/ml antib. ◨ 0.25µg/ml antib.
▤ 0.5µg/ml antib.  ■ 1µg/ml antib.     □ 2µg/ml antib.
▨ 4µg/ml antib.   ▨ 8µg/ml antib.     ⊠ 16µg/ml antib.
⊠ 32µg/ml antib.  ▨ 64µg/ml antib.    ▦ 128µg/ml antib.
□ 256µg/ml antib.

**Fig. 11B**

**Fig. 11 C**

**Fig. 11 D**

**EUROPEAN SEARCH REPORT**

Application Number

EP 19 18 7294

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2018/292324 A1 (VERMA RAVI [US] ET AL) 11 October 2018 (2018-10-11) * whole document, in particular [0017], [0034], [0356],[0413], fig. 10, 32A,32B * | 1-15 | INV. G16H20/10 G01N33/52 |
| A | JOHANNA KIRCHHOFF ET AL: "Simple Ciprofloxacin Resistance Test and Determination of Minimal Inhibitory Concentration within 2 h Using Raman Spectroscopy", ANALYTICAL CHEMISTRY, vol. 90, no. 3, 6 February 2018 (2018-02-06), pages 1811-1818, XP055661952, US ISSN: 0003-2700, DOI: 10.1021/acs.analchem.7b03800 | 1-15 | |
| A | BERTRAND CINQUIN ET AL: "Microspectrometric insights on the uptake of antibiotics at the single bacterial cell level", SCIENTIFIC REPORTS, vol. 5, no. 1, 1 December 2015 (2015-12-01), XP055661960, DOI: 10.1038/srep17968 * the whole document * | 1-15 | |

TECHNICAL FIELDS SEARCHED (IPC)

G16H
G01N

The present search report has been drawn up for all claims

| Place of search | Date of completion of the search | Examiner |
|---|---|---|
| Munich | 27 January 2020 | Lüdemann, Susanna |

EPO FORM 1503 03.82 (P04C01)

## ANNEX TO THE EUROPEAN SEARCH REPORT
## ON EUROPEAN PATENT APPLICATION NO.

EP 19 18 7294

This annex lists the patent family members relating to the patent documents cited in the above-mentioned European search report.
The members are as contained in the European Patent Office EDP file on
The European Patent Office is in no way liable for these particulars which are merely given for the purpose of information.

27-01-2020

| Patent document cited in search report | Publication date | Patent family member(s) | | Publication date |
|---|---|---|---|---|
| US 2018292324 A1 | 11-10-2018 | EP | 3602065 A1 | 05-02-2020 |
| | | US | 2018292324 A1 | 11-10-2018 |
| | | WO | 2018175346 A1 | 27-09-2018 |

EPO FORM P0459

For more details about this annex : see Official Journal of the European Patent Office, No. 12/82

**EP 3 767 631 A1**